# EUROPEAN PATENT SPECIFICATION

(11) **EP 1 274 717 B1**
(45) Date of publication and mention of the grant of the patent: **27.02.2008**
(21) Application number: 01926716.0
(22) Date of filing: 06.04.2001
(51) Int. Cl.: C07H 21/04, C07K 14/775

(54) **COMPOUNDS AND METHODS FOR LOWERING CHOLESTEROL LEVELS WITHOUT INDUCING HYPERTRIGLYCERIDEMIA**
VERBINDUNGEN UND METHODEN ZUR SENKUNG DES CHOLESTERINSPIEGELS OHNE INDUKTION VON HYPERTRIGLYCERIDÄMIE
COMPOSES ET METHODES PERMETTANT DE REDUIRE LES TAUX DE CHOLESTEROL SANS INDUIRE UNE HYPERTRIGLYCERIDEMIE

(30) Priority: 06.04.2000 US 544386; 04.10.2000 US 679088; 05.04.2001 US 827854
(43) Date of publication of application: 15.01.2003
(73) Proprietor: KOS PHARMACEUTICALS, INC., Miami, FL 33131 (US); Trustees of Boston University, Boston, MA 02215 (US)
(72) Inventor: ZANNIS, Vassilis, I., Newton, MA 02159 (US); KYPREOS, Kyriakos, E., GR-851 01 Rhodes (GR)
(74) Representative: Schnappauf, Georg
(86) International application number: PCT/US2001/011358
(87) International publication number: WO 2001/077136

(56) References cited:
- US-A- 5 958 684
- WESTERLUND JOHN A ET AL: "Discrete carboxyl-terminal segments of apolipoprotein E mediate lipoprotein association and protein oligomerization" JOURNAL OF BIOLOGICAL CHEMISTRY, vol. 268, no. 21, 1993, pages 15745-15750, XP002279084 ISSN: 0021-9258
- DONG L-M ET AL: "HUMAN APOLIPOPROTEIN E ROLE OF ARGININE 61 IN MEDIATING THE LIPOPROTEIN PREFERENCES OF THEE3 AND E4 ISOFORMS" JOURNAL OF BIOLOGICAL CHEMISTRY, THE AMERICAN SOCIETY OF BIOLOGICAL CHEMISTS, INC.,, US, vol. 269, no. 35, 2 September 1994 (1994-09-02), pages 22358-22365, XP001008709 ISSN: 0021-9258
- DONG L-M ET AL: "HUMAN APOLIPOPROTEIN E4 DOMAIN INTERACTION ARGININE 61 AND GLUTAMIC ACID 255 INTERACT TO DIRECT THE PREFERENCEFOR VERY LOW DENSITY LIPOPROTEINS" JOURNAL OF BIOLOGICAL CHEMISTRY, THE AMERICAN SOCIETY OF BIOLOGICAL CHEMISTS, INC.,, US, vol. 271, no. 32, 9 August 1996 (1996-08-09), pages 19053-19057, XP001018932 ISSN: 0021-9258
- TSUKAMOTO KAZUHISA ET AL: "Liver-directed gene transfer and prolonged expression of three major human ApoE isoforms in ApoE-deficient mice" JOURNAL OF CLINICAL INVESTIGATION, vol. 100, no. 1, 1997, pages 107-114, XP002279247 ISSN: 0021-9738
- DUAN H ET AL: "Sterols and inhibitors of sterol transport modulate the degradation and secretion of macrophage ApoE: requirement for the C-terminal domain" BIOCHIMICA AND BIOPHYSICA ACTA. MOLECULAR AND CELL BIOLOGY OF LIPIDS, ELSEVIER, AMSTERDAM, NL, vol. 1484, no. 2-3, 10 April 2000 (2000-04-10), pages 142-150, XP004277326 ISSN: 1388-1981
- LI XIAOPING ET AL: "Domains of apoE required for binding to apoE receptor 2 and to phospholipids: Implications for the functions of apoE in the brain." BIOCHEMISTRY, vol. 42, no. 35, 9 September 2003 (2003-09-09), pages 10406-10417, XP002279085 ISSN: 0006-2960 (ISSN print)
- KYPREOS KYRIAKOS E ET AL: "Molecular mechanisms of type III hyperlipoproteinemia: The contribution of the carboxy-terminal domain of ApoE can account for the dyslipidemia that is associated with the E2/E2 phenotype." BIOCHEMISTRY, vol. 42, no. 33, 26 August 2003 (2003-08-26), pages 9841-9853, XP002279086 ISSN: 0006-2960 (ISSN print)
- KYPREOS KYRIAKOS E ET AL: "The amino-terminal 1-185 domain of apoE promotes the clearance of lipoprotein remnants in vivo. The carboxy-terminal domain is required for induction of hyperlipidemia in normal and apoE-deficient mice" BIOCHEMISTRY, vol. 40, no. 20, 22 May 2001 (2001-05-22), pages 6027-6035, XP002279087 ISSN: 0006-2960
- KYPREOS KYRIAKOS E ET AL: "Domains of apolipoprotein E contributing to triglyceride and cholesterol homeostasis in vivo: Carboxyl-terminal region 203-299 promotes hepatic very low density lipoprotein-triglyceride secretion" JOURNAL OF BIOLOGICAL CHEMISTRY, vol. 276, no. 23, 8 June 2001 (2001-06-08), pages 19778-19786, XP002279088 ISSN: 0021-9258
- KYPREOS KYRIAKOS E ET AL: "Analysis of the structure and function relationship of the human apolipoprotein E in vivo, using adenovirus-mediated gene transfer" FASEB JOURNAL, vol. 15, no. 9, July 2001 (2001-07), pages 1598-1600, XP002279089 ISSN: 0892-6638
- MATSUSHIMA ET AL.: 'Primary structure of guinea pig apolipoprotein E' NUCLEIC ACIDS RES. vol. 18, no. 1, January 1990, page 202, XP002944036
- LUO ET AL.: 'Structure and expression of dog apolipoprotein A-I, E and C-I mRNAs: Implications for the evolution and functional constraints of apolipoprotein structure' J. LIPID RES. vol. 30, November 1989, pages 1735 - 1746, XP002944037
- VERMA ET AL.: 'Gene therapy: Promises, problems and prospects' NATURE vol. 389, 18 September 1997, pages 239 - 242, XP002944039
- RUDINGER J., PARSONS J.A. ED.: 'Characteristics of the amino acids as components of a peptide hormone sequence', June 1976, UNIVERSITY PARK PRESS, LONDON, GB article 'Peptide Hormones', pages 1 - 7, XP002919149

## Description

### Statement as to Federally Sponsored Research

This invention was funded in part by National Institutes of Health grant AG12717. The government may have certain rights in the invention.

### Background of the Invention

As a ligand that promotes the recognition and catabolism of apolipoprotein E (apoE)-containing lipoproteins by cell receptors, apoE is an important component of the cholesterol transport system (Innerarity and Mahley, Biochemistry 17:1440-1447, 1978; Herz and Willnow, Curr. Opin. Lipidol. 6:97-103, 1995; Wolf et al., Am. J. Pathol. 141:37-42, 1992; Kim et al., J. Biol. Chem. 271:8373-8380, 1996; Takahashi et al., Proc. Natl. Acad. Sci. USA 89:9252-9256, 1992; Mahley et al., Curr. Opin. Lipidol. 10:207-217, 1999; Wardell et al., J. Clin. Invest. 80:483-490, 1987; Cohn et al., Vas. Biol. 16:149-159, 1996; Chait et al., Metabolism 27:1055-1066, 1978; Huang et al., Proc. Natl. Acad. Sci. USA 91:1834-1838, 1994; Huang et al., Arterioscler. Thromb. Vasc. Biol. 17:2010-2019, 1997). Heparin sulfate proteoglycans may also be involved in this process (Cullen et al., J. Clin. Invest. 101:1670-1677, 1998; Linton et al., Science 267:1034-1037, 1995; Fazio et al., Proc. Natl. Acad. Sci. USA 95:4647-4652,1997; Huang et al., J. Biol. Chem. 273:26388-26393, 1998; van Dijk et al., J. Lipid Res., 40:336-344, 1999; Huang et al., Arterioscler. Thromb. Vasc. Biol, 19:2952-2959, 1999; Salah et al., J. Lipid Res. 38:904-912,1997; Ji et al., J. Biol. Chem. 268:10180-10187, 1993; Ji et al., J. Biol. Chem. 269:13421-13428, 1994; Ji et al., J. Lipid Res. 36:583-592, 1995; Ji et al., J. Biol. Chem. 269:2764-2772, 1994). Mutations in apoE that prevent its binding to the LDL receptor and possibly other receptors and heparin sulfate proteoglycans are associated with type III hyperlipoproteinemia and premature atherosclerosis (Mahley et al., Curr. Opin. Lipidol. 10:207-217, 1999; Dong et al., Nature Struc. Biol. 3:718-722, 1996; Wardell et al., J. Clin. Invest. 80:483-490, 1987; Rall et al., J. Clin. Invest. 83:1095-1101, 1989; Mann et al., Biochim. Biophys. Acta 1005:239-244, 1989; Wardell et al., J. Biol. Chem. 264:21205-21210, 1989; van den Maagdenberg et al., Biochem. Biophys. Res. Commun. 165:851-857, 1989; Smit et al.,. J. Lipid Res. 31:45-53, 1990; Ghiselli et al., Science 214:1239-124, 198; Lalazar et al., J. Biol. Chem. 263:3542-3545, 1988; Weisgraber et al., J. Biol. Chem, 258:12348-12354, 1983; Innerarity et al., J. Biol. Chem. 258:12341-12347, 1983).

ApoE is a 34.2 kDA protein synthesized by the liver and various peripheral tissues, including kidney, adrenal gland, astrocytes, and reticuloendothelial cells. ApoE is synthesized as a precursor with a 18-amino acid signal peptide. After the intracellular cleavage of the signal peptide, apoE is glycosylated with carbohydrate chains containing sialic acid and secreted as sialo apoE. It is subsequently desialated in plasma (see Zannis et al., Adv. Hum. Genet. 21:145-319, 1993; Zannis et al., J. Biol. Chem. 259:5495-5499, 1984; and Zannis et al., J. Biol. Chem. 261:13415-13421, 1986).

As would be readily apparent to one skilled in the art, the amino acid numbering for the apoE proteins used herein refers to the mature protein after cleavage of the signal peptide. The amino acids of the signal peptide are numbered -18 to -1, with -18 referring to the amino-terminal residue of the preprotein (Karathansis et al., "Nucleotide and Corresponding Amino Acid Sequences of Human apoA-1, apoA-11, apoC1, apoC11, apoC111, and apoE cDNA clones" In Biochemistry and Biology of Plasma Proteins, Scanu and Spector, eds., Marcel Dekker, New York, vol. 11, pp. 475-493, 1985).

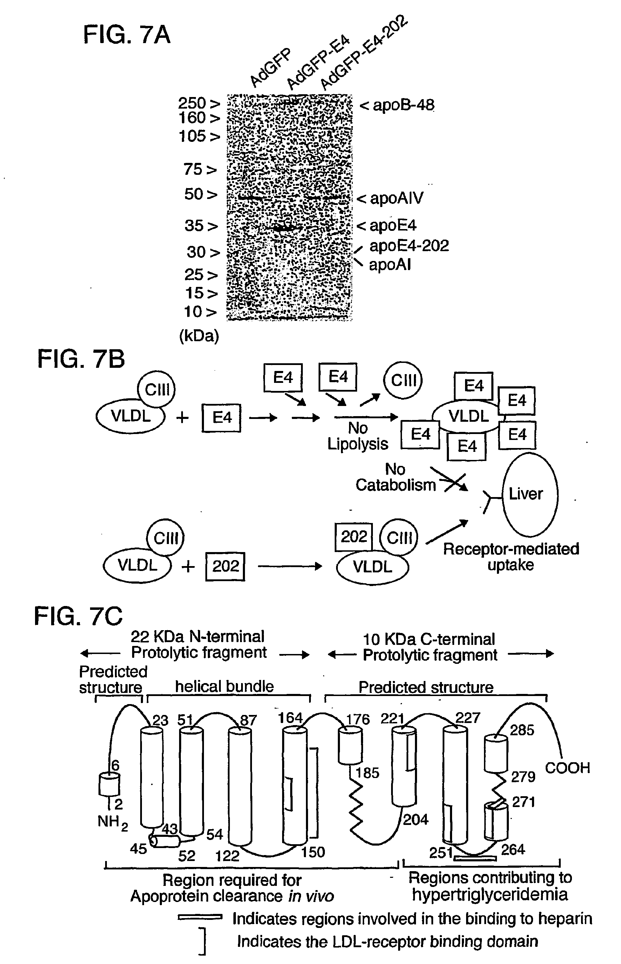

Several domains of apoE have been described which are presumably involved in receptor binding (He et al., Proc. Natl. Acad. Sci. USA 95:2509-2514, 1998; Lalazar, *supra;* Weisgraber, *supra* (1983); Innerarity et al., J. Biol. Chem. 258:12341-12347, 1983; Ji et al., J. Lipid Res. 36:583-592, 1995; Rall, et al., Proc. Natl. Acad. Sci. USA 79, 4696-4703, 1982; Westerlund et al., J. Biol. Chem, 268:15745-15750, 1993; Wilson et al., Structure 2:713-718, 1994; Wilson et al., Science 252:1817-1822, 1991; Mahley, Biochim. Biophys Acta 575:81-91, 1979), heparin binding (Lalazar, *supra;* Fan, *supra;* Salah, *supra;* Ji, *supra* (1993)), lipid binding and lipoprotein binding (Cohn, *supra;* Huang et al., J. Biol. Chem. 273:26388-26393, 1998; Salah, *supra;* Ji, *supra* (J. Biol. Chem. 269, 1994); Ji, *supra* (1995); Ji, *supra* (J. Biol. Chem. 289, 1994)) (Fig. 7C). The receptor binding domain is found between residues 136-152 while neighboring residues may also indirectly affect receptor binding.

Dong et al. describe the role of arginine 61 in mediating the lipoprotein preferences of the E3 and E4 isoforms (J. Biol. Chem. 1994, Vol. 269(35):22358-22365). In another publication Dong et al. further discuss apolipoprotein E4 domain interaction (J. Biol. Chem. 1996, Vol. 271(32):19053-19057). Tsukamoto et al. mention the liver-directed gene transfer and prolonged expression of three major human ApoE isoforms in ApoE-deficient mice (Journal of Clinical Investigation 1997, Vol. 100(1):107-1148).

One of the heparin binding domains overlaps with the receptor binding domain between residues 140-150, while two other heparin binding domains were reported between residues 211-218 and 243-272 (Weisgraber et al., J. Biol. Chem. 261:2068-2076, 1986; Cardin, *supra).* Previous studies indicate that the 140-150 domain is directly involved in the binding of apoE-containing lipoproteins to heparin sulfate proteoglycans, and their subsequent internalization with or without the participation of LRP (LDL receptor related protein) (Herz, *supra;* Mahley, *supra;* Fazio, *supra;* van Dijk, *supra;* Huang, *supra* (1999); Ji, *supra* (J. Biol. Chem. 289, 1995); Cardin, *supra;* Dong, et al., J. Biol. Chem. 269:22358-22365, 1994). The prevailing concept regarding lipid and lipoprotein binding is that the region of apoE between residues 244-266 contributes to the binding of apoE to lipids and lipoproteins, whereas the amino terminal region of apoE lacks the determinants required for association with lipoproteins (He, *supra;* Dong, *supra* (1994)).

There are three common alleles that encode apoE in humans. The three alleles designated e4, ε3, and ε2 give rise to three homozygous phenotypes (*i.e.,* E4/E4, E3/E3, and E2/E2) and three heterozygous phenotypes (*i.e*., E4/E3, E3/E2, and E4/E2) (Zannis and Breslow, Biochemistry 20:1033-1041, 1982; Zannis et al., Am. J. Hum. Genet. 33:11-24, 1981). The three different human apoE isoproteins, apoE4, apoE3, and apoE2, result from mutations at amino residues 112 and 158. ApoE4 contains Arg at position 112 and Arg at position 158. ApoE3 contains Cys at position 112, and Arg at position 158. ApoE2 contains Cys at positions 112 and 158.

In addition to the common apoE alleles, there are three rare apoE alleles (apoE1, apoE2*, and apoE2**). Compared to the other apoE proteins, apoE1 has Asp at position 127 instead of Gly and Cys at position 158 instead of Arg. ApoE2* has Cys at position 145 instead of Arg, and apoE2** has Gln at position 146 instead of Lys (Karathanasis *et al., supra*).

Compelling evidence on the role of apoE in cholesterol homeostasis was established unequivocally by studies of human patients and animal models with apoE deficiency or defective apoE forms (Schaefer et al., J. Clin. Invest. 78:1206-1219, 1986; Cladaras et al., J. Biol. Chem. 262:2310-2315, 1987; Plump et al., Cell 71:343-353, 1992; Zhang et al., Science 2588:468-471, 1992; Reddick et al., Arterioscler. Thromb. 14:141-147, 1994; Vanden Maagdenberg et al., J. Biol. Chem. 268:10540-10545, 1993; Fazio et al., J. Clin. Invest. 92:1497-1503, 1993; Fazio et al., J. Lipid Res. 35:408-416, 1994; Fazio et al., Arterioscler. Thromb. 14:1873-1879,1994; Vlismen et al., J. Biol. Chem. 271:30595, 1996). These studies show that apoE is required for the clearance of cholesterol ester-rich lipoprotein remnants which float in the VLDL and IDL region (Plump et al., Cell 71:343-353, 1992; Zhan et al., Science 2588:468-471, 1992; Reddick et al., Arterioscler. Thromb. 14:141-147, 1994; Vanden Maagdenberg et al., J. Biol. Chem. 268:10540-10545, 1993; Fazio et al., J. Clin. Invest. 92:1497-1503,1993; Fazio et al., J. Lipid Res. 35:408-416, 1994; Fazio et al., Arterioscler. Thromb. 14:1873-1879, 1994; van Vlijmen et al., J. Biol. Chem. 271:30595-3062, . 1994; Cohn et al., Arterioscler. Thromb. Vas. Biol. 16:149-159, 1996; Chait et al., Metabolism 27:1055-1066, 1978). The accumulation of such remnants in plasma is associated with premature atherosclerosis (Schaefer et al., J. Clin. Invest. 78:1206-1219, 1986; Plump et al., Cell 71:343-353, 1992; Zhang et al., Science 2588:468-471, 1992; Reddick et al., Arterioscler. Thromb. 14:141-147, 1994).

Other studies highlight the importance of apoE in cholesterol efflux and show that apoE-containing lipoprotein particles with γelectrophoretic mobility (γLp-E) are very effective in removing excess cholesterol from cholesterol-loaded macrophages, thus contributing to cell and tissue cholesterol homeostasis (Huang et al., Proc. Natl. Acad. Sci. USA 91:1834-1838, 1994; Huang et al., Arterioscler. Thromb. Vasc. Biol. 17:2010-2019, 1997; Zhu et al., Proc. Natl. Acad. Sci. USA 95:7585-7590, 1998; Cullen et al., J. Clin. Invest. 101:1670-1677, 1998). The involvement of apoE in cholesterol efflux may explain why, when expressed locally in macrophages or endothelial cells, apoE protects from atherosclerosis (Linton et al., Science 267:1034-1037,1995; Fazio et al., Proc. Natl. Acad. Sci. USA 95:4647-4652, 1997; Shimano et al., J. Clin. Invest. 95:469-476, 1995).

Recent studies in humans and in transgenic animal models have indicated that apoE may have other functions relevant to plasma triglyceride homeostasis. Such studies show that increases in apoE levels inhibit lipolysis of triglyceride-rich lipoproteins, resulting in hypertriglyceridemia (Cohn et al., Arterioscler. Thromb. Vas. Biol. 16:149-159, 1996; Chait et al., Metabolism 27:1055-1066, 1978; Huang et al., J. Biol. Chem. 273:26388-26393,1998; Ehnholm et al., Proc. Natl. Acad. Sci. USA 81:5566-5570, 1984; Huang et al., J. Biol. Chem. 273:17483-17490, 1998; Rensen et al., J. Biol. Chem. 271:14791-14799, 1996; Jong et al., Biochem. J. 328:745-750, 1997, van Dijk et al., JLR, 40:336-344, 1999; Salah. et al., J. Lipid Res. 38:904-912, 1997; Ji et al., J. Lipid Res. 36:583-592, 1995; Ji et al., J. Biol. Chem. 289:2784-2772, 1994; Rall et al., Proc. Natl. Acad. Sci. USA 79,4696-4700,1992; Weisgraber, *supra* (1983); Cardin et al., Biochem. Biophys. Res. Commun. 134:783-789, 1986; Dong, *supra* (1994); Westerlund, *supra;* Wilson et al., Structure 2:713-718, 1994). Lipolysis of VLDL in vitro could be partially restored by the addition of apoCII (Huang et al., J. Biol. Chem. 273:26388-26393, 1998; Huang et al., J. Biol. Chem. 273:17483-17490, 1998). This apoE function may result in high triglyceride levels in the human population (Cohn, *supra;* Chait, *supra;* Salah, *supra*; Ji, *supra* (1995)). Overexpression of apoE also stimulates hepatic VLDL triglyceride production *in vivo* (Huang, *supra* (1999)) and in cell cultures (Huang et al., J. Biol. Chem. 273:26388-26393, 1998), possibly by promoting the assembly and/or secretion of apoB-containing lipoproteins. This possible participation of apoE in VLDL assembly and secretion might proceed through the mobilization of membrane lipids (Huang et al., J. Biol. Chem. 273:26388-26393 40, 1998; Huang et al., Arterioscler. Thromb. Vasc. Biol. (in press), 1999; Fan et al., J. Clin. Invest. 101:2151-2164, 1998; Kulpers et al., J. Clin. Invest 100:2915-2922.893. 1997; Rall et al., Proc. Natl. Acad. Sci. USA 79,4696-4700,1982). In contrast, lack of apoE is associated with decreased VLDL triglyceride secretion (Kulpers et al., J. Clin. Invest. 100:2915-2922, 1997). The injection of two ¹²⁵I truncated apoE forms extending from residues 1-191 and 1-244 respectively, resulted in their fast and efficient removal from plasma (Westerlund, *supra).*

Despite the beneficial effects of apoE in cholesterol homeostasis, the therapeutic value of apoE in gene therapy approaches remains very limited, due to the severe hypertriglyceridemia and VLDL accumulation that may be triggered by apoE overexpression in animal studies. A therapy is needed that will lower cholesterol levels without inducing hypertriglyceridemia.

### Summary of the Invention

In a first aspect, the invention relates to the use of a polypeptide comprising a region of at least 150 amino acids having at least 80% sequence identity to the corresponding region of amino acids 1-259 of a mature, native, human apoE, wherein the polypeptide does not comprise the carboxyl-terminal 260-299 residues of said apoE, for the preparation of a pharmaceutical composition for lowering cholesterol, delaying the onset of atherosclerosis, or treating atherosclerosis in a mammal without inducing hypertriglyceridemia, wherein said polypeptide is administered to said mammal and said polypeptide, when administered to or expressed in a mammal, is capable of lowering the total serum cholesterol level without inducing hypertriglyceridemia.

In a second aspect, the invention relates to the use of a nucleic acid encoding a polypeptide comprising a region of at least 150 amino acids having at least 80% sequence identity to the corresponding region of amino acids 1-259 of a mature, native, human apoE polypeptide, wherein the nucleic acid does not encode the carboxyl-terminal 260-299 residues of said apoE, for the preparation of a pharmaceutical composition for lowering cholesterol, delaying the onset of atherosclerosis, or regressing atherosclerosis in a mammal without inducing hypertriglyceridemia, wherein said nucleic acid is administered to or expressed in said mammal and the nucleic acid, when administered to or expressed in a mammal, lowers the total serum cholesterol level without inducing hypertriglyceridemia.

Preferably, this decrease in cholesterol level is at least 10%, 20%, 30%, 50%, 70%, or 90%. By "hypertriglyceridemia" is meant an increase in triglyceride concentration by more than 15%. Cholesterol and triglyceride levels are determined using the standard assays described herein.

The polypeptides and nucleic acids used according to the invention can be administered to or expressed in a mammal, preferably a human patient, to lower cholesterol, delay the onset of atherosclerosis, or treat atherosclerosis without inducing hypertriglyceridemia. Particularly suitable patients are those who lack an endogenous, normally functioning apoE gene or who are at risk for developing atherosclerosis due to a defect in remnant removal that results in the accumulation of lipoprotein remnants in the bloodstream. Other particularly suitable patients have a lower than normal level of LDL receptor protein. For example, the patients may have a mutation in the regulatory, promoter, or coding sequence for the LDL receptor that reduces or prevents expression of an endogenous, full length LDL receptor. Alternatively, the patient may have a missense mutation that reduces an activity of the encoded LDL receptor, such as the binding of the LDL receptor to apoE. Preferably, the polypeptide is administered, with a pharmaceutically acceptable carrier substance, intramuscularly, intravenously, or subcutaneously. Preferably, the polypeptide is directly delivered to an atherosclerotic plaque and/or the surrounding tissue in the artery. In other preferred embodiments, the polypeptide is provided as a result of gene therapy, or as a result of bone marrow transplantation. Preferably, the nucleic acids used according to the invention are administered intravenously in combination with a liposome and protamine. In preferred embodiments, the nucleic acid is administered to, or expressed in, the liver, vascular wall, or atherosclerotic plaque of the mammal. In other preferred embodiments, the nucleic acid is directly delivered to the site of an atherosclerotic lesion using a recombinant virus. In other preferred embodiments, the nucleic acid is provided as a result of genetic manipulation of a human fetus or bone marrow transplantation. In other preferred embodiments, the nucleic acid is operably linked to a promoter and contained in an expression vector, e.g. a plasmid or a recombinant viral vector, such as an adenoviral, adeno-associated viral, retroviral, lentiviral, herpes viral vector, or baculovirus-based system.

In a further aspect, the invention provides a polypeptide that, when administered to or expressed in a mammal, lowers the total serum cholesterol level without inducing hypertriglyceridemia and that has a sequence identical to residues 1-185, 1-202, 1-229, or 1-259 of mature apoE4 (SEQ ID No. 1), apoE3 (SEQ ID No. 2), apoE2 (SEQ ID No. 3), apoE1 (SEQ ID No. 4), apoE2* (SEQ ID No. 5), or apoE2** (SEQ ID No. 6). In other embodiments, the polypeptide has an amino acid sequence identical to residues 1-203, 1-220, 1-247, or 1-277 of an apoE preprotein of any one of SEQ ID NOs. 14-19.

Another aspect of the invention relates to a nucleic acid having a sequence encoding residues 1-185,1-202,1-229, or 1-259 of mature apoE4 (SEQ ID No. 1), apoE3 (SEQ ID No. 2), apoE2 (SEQ ID No. 3), apoE1 (SEQ ID No. 4), apoE2* (SEQ ID No. 5), or apoE2** (SEQ ID No. 6) or the first 203, 220, 247, or 277 residues of an apoE preprotein selected from the group consisting of apoE4 (SEQ ID No. 14), apoE3 (SEQ ID No. 15), apoE2 (SEQ ID No. 16), apoE1 (SEQ ID No. 17), apoE2* (SEQ ID No. 18), or apoE2** (SEQ ID No. 19), which contain an 18 amino acid N-terminal signal sequence in addition to the sequence of the mature apoE protein.

In another aspect, the invention features a pharmaceutical composition that includes a polypeptide of the present invention admixed with a pharmaceutically acceptable carrier substance.

In yet another aspect, the invention provides a recombinant DNA molecule that includes a nucleic acid of the present invention operatively linked to a promoter.

The invention takes advantage of the cholesterol lowering property of apoE, while avoiding its induction of hyperglyceridemia.

Other features and advantages of the invention will be apparent from the following description of the preferred embodiments thereof, and from the claims.

### Brief Descriptions of the Drawings

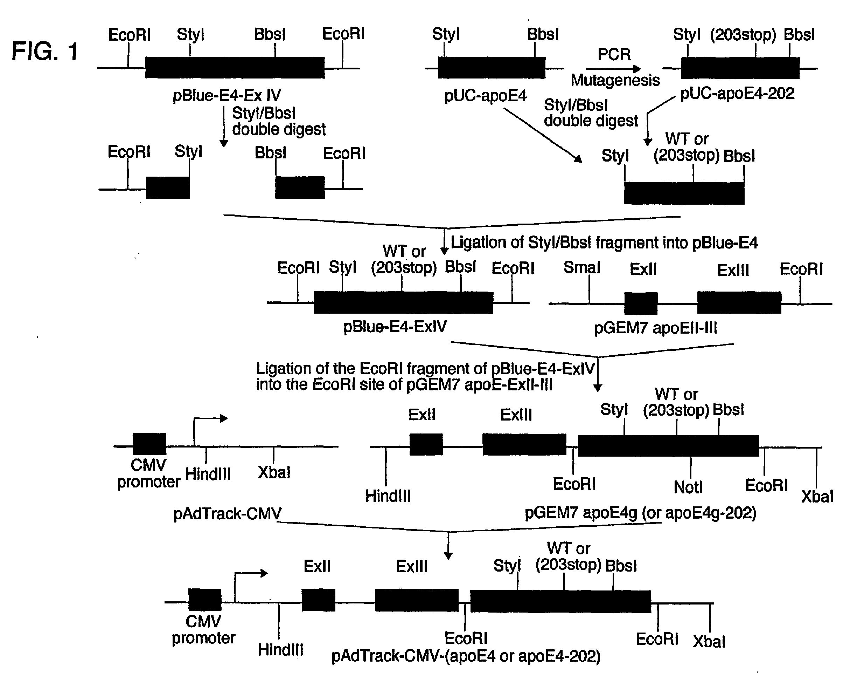

Fig.1 is a schematic representation of the steps leading to the production of the apoE4-202 plasmids of the invention. The same method was used for generation of the corresponding apoE4-185, apoE4-229, and apoE4-259 plasmids.

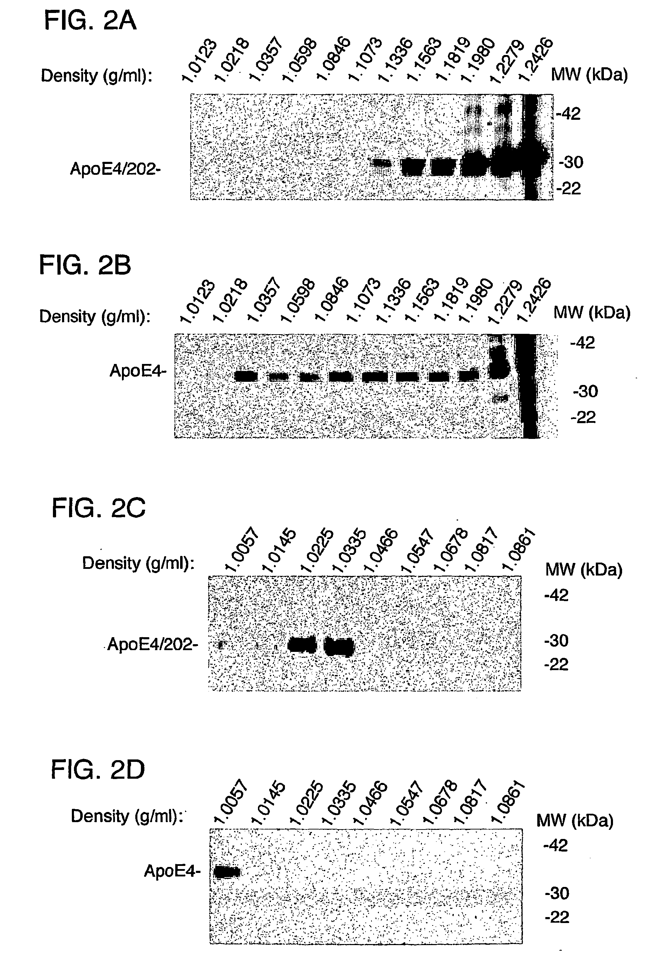

Figs. 2A and 2B are pictures of protein gels which demonstrate the ability of apoE4 and the truncated apoE4-202 to associate with lipoproteins in the density = 1.04 to 1.21 g/ml fractions. Figs. 2C and 2D are pictures of protein gels which demonstrate the ability of apoE4 and apoE4-202 to associate with VLDL particles.

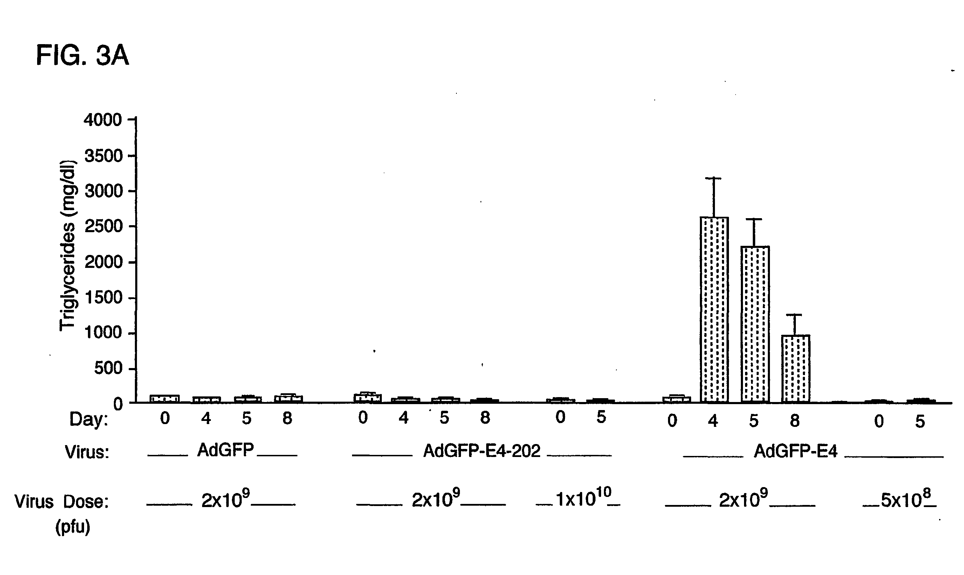

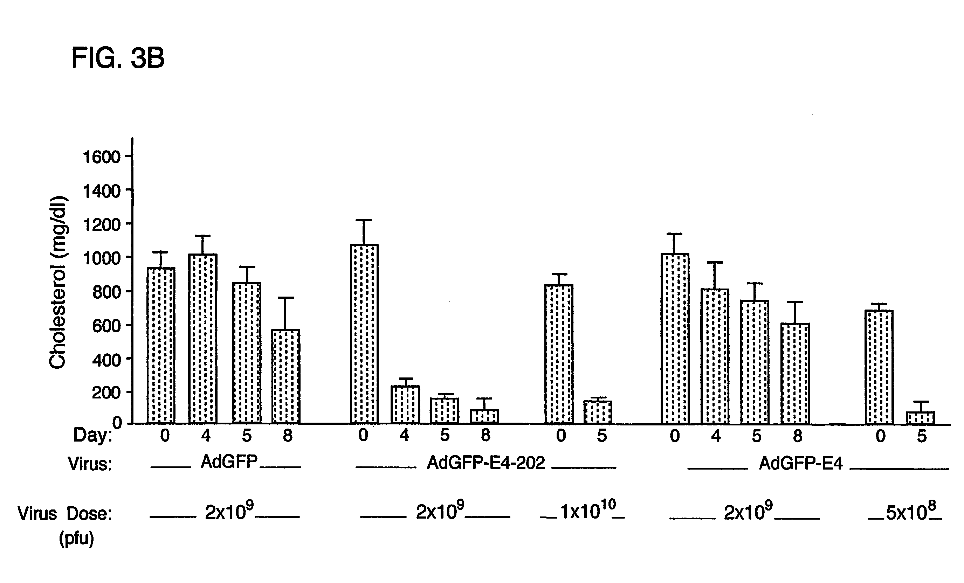

Fig. 3A is a bar graph showing that recombinant adenoviruses expressing apoE4 increase the triglyceride levels in apoE-deficient mice. In contrast, a recombinant adenovirus expressing apoE4-202 does not cause this increase. Fig. 3B is a bar graph showing that recombinant adenoviruses expressing apoE4-202 produce a greater decrease in cholesterol levels in apoE-deficient mice than the corresponding viruses expressing full length apoE.

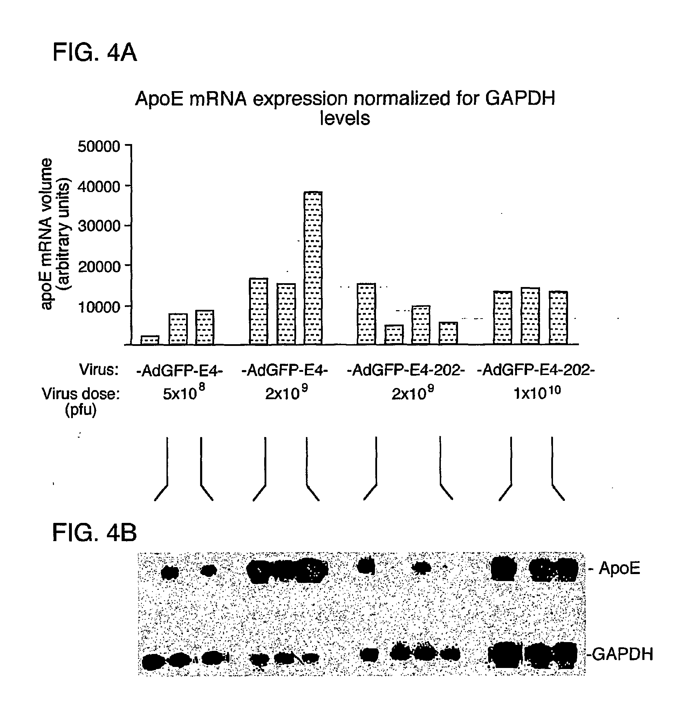

Figs. 4A is a bar graph and Fig. 4B is a picture of a gel demonstrating that recombinant adenoviruses expressing apoE4 or apoE4-202 synthesize comparable amounts of mRNA.

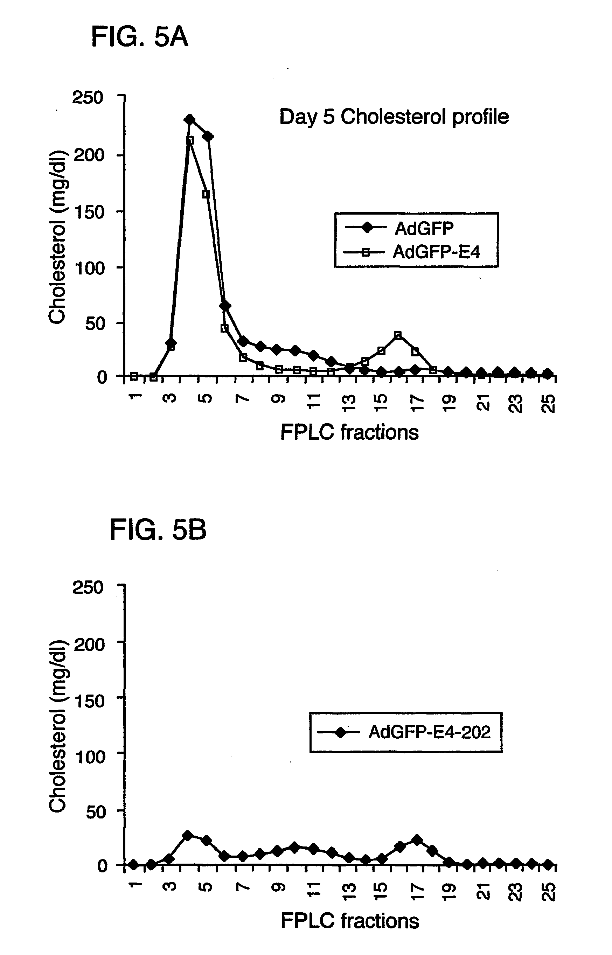

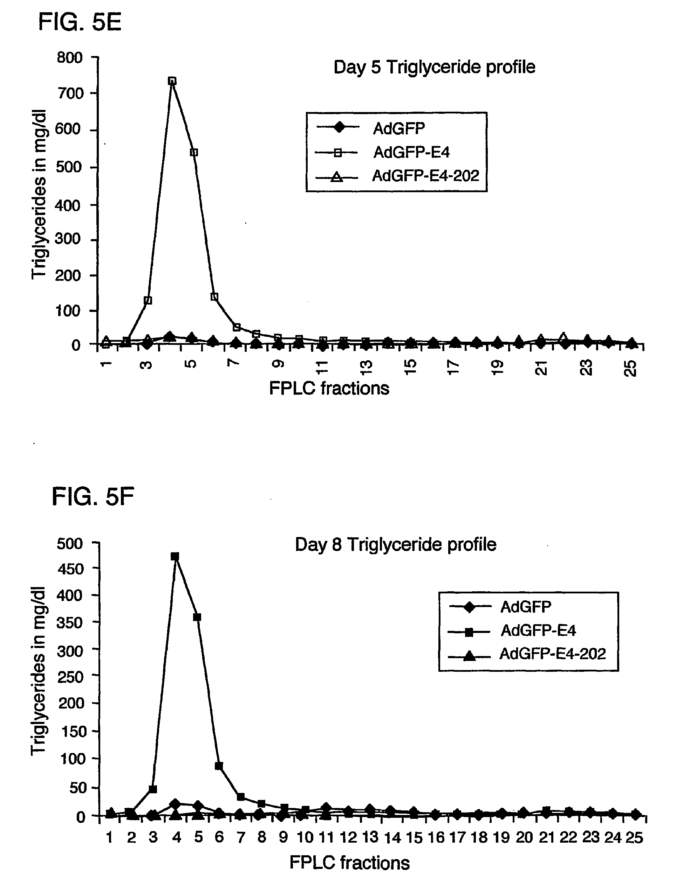

Figs. 5A-5D are graphs showing that apoE-deficient mice infected with AdGFP-E4-202 had lower cholesterol levels on days 5 and 8 after infection than those infected with AdGFP-E4 or the AdGFP control where most of the cholesterol was found in the VLDL region. Figs. 5E and 5F are graphs showing the increase in VLDL-triglyceride levels in mice on days 5 and 8 after infection with AdGFP-E4. In contrast, infection with AdGFP-E4-202 did not increase triglyceride levels.

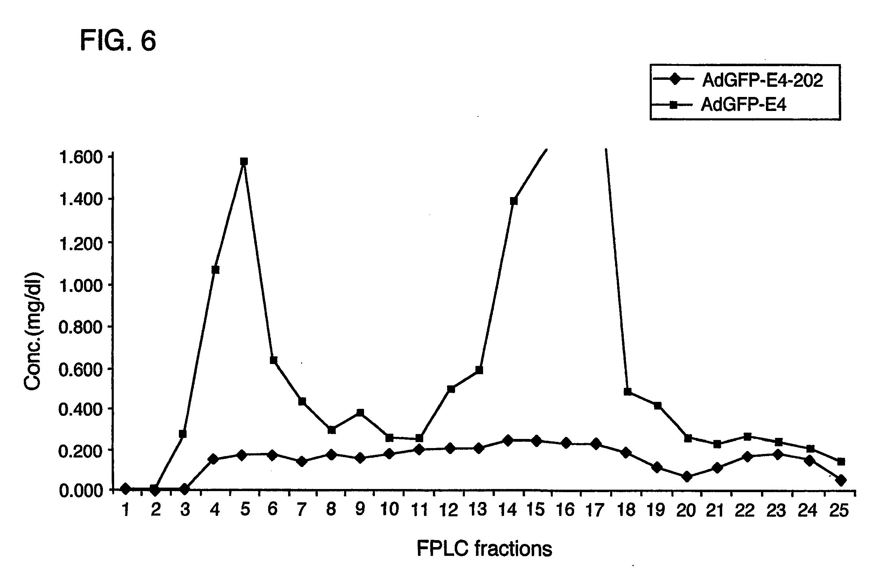

Fig. 6 is a graph showing that the plasma of apoE-deficient mice infected with AdGFP-E4 or AdGFP-E4-202 have similar levels of lipid-free apoE protein (fractions 22-25). In the plasma of mice infected with AdGFP-E4, approximately 50% of the total apoE is distributed in the HDL fractions and 25% in the VLDL fractions, with the remaining protein distributed across the other FPLC fractions, as measured by ELISA. In apoE-deficient mice infected with AdGFP-E4-202, the truncated apoE protein is present at a lower level than the full length apoE in mice infected with AdGFP-E4 and is uniformly distributed in all lipoprotein fractions.
Fig. 7A is a picture of a protein gel illustrating that in apoE-deficient mice infected with the recombinant adenovirus expressing apoE4, the apoE4 displaces other proteins from the VLDL density particles. No displacement of other proteins from the VLDL density particles is detected in mice infected with recombinant adenovirus expressing apoE4-202. Fig. 7B is a schematic illustration showing how the displacement of other proteins from the VLDL density particles by apoE4 could be the cause of hypertriglyceridemia. Fig. 7C is a schematic illustrations of the putative domains of apoE.

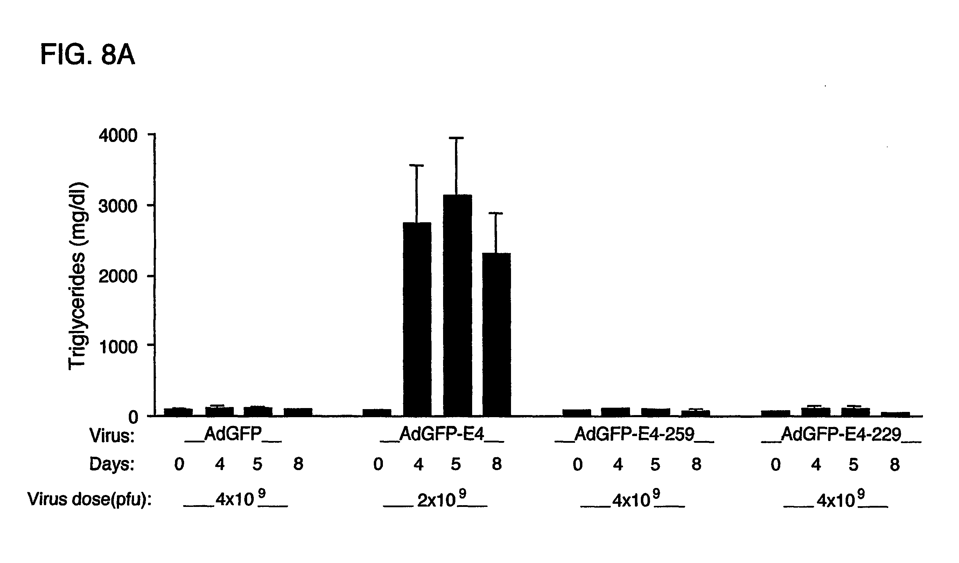

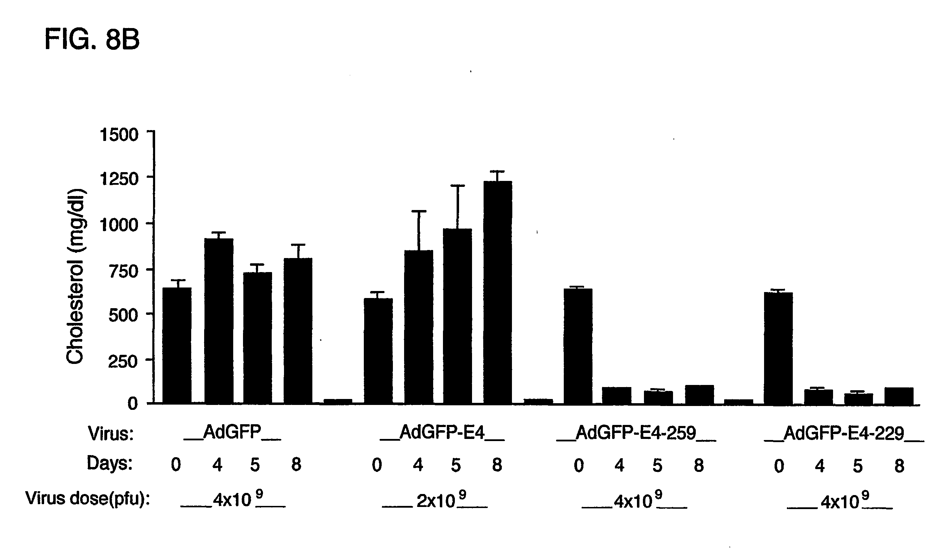

Figs. 8A and 8B are bar graphs showing an *in vivo* time course analysis of serum triglyceride (Fig. 8A) and cholesterol levels (Fig. 8B) in apoE-deficient mice infected with an AdGFP control (no apoE), AdGFP-E4, AdGFP-E4-229, or AdGFP-E4-259. Infection of apoE-deficient mice with a dose of 4x10⁹ pfu of the adenoviruses expressing the truncated E4-229 or E4-259 forms significantly reduced the level of cholesterol without increasing the level of serum triglycerides. In contrast, infection with a dose of 2x10⁹ pfu of the adenovirus expressing wild-type E4 does not appear to reduce cholesterol and causes a dramatic increase in serum triglyceride levels. The control virus AdGFP does not appear to have any effects on the basal (day 0) cholesterol and triglyceride levels of the apoE-deficient mice.

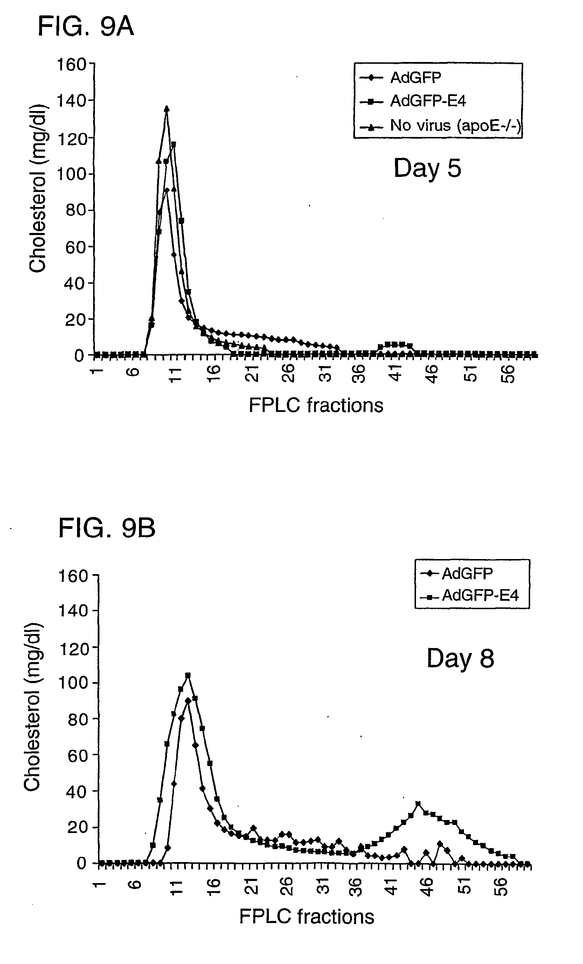

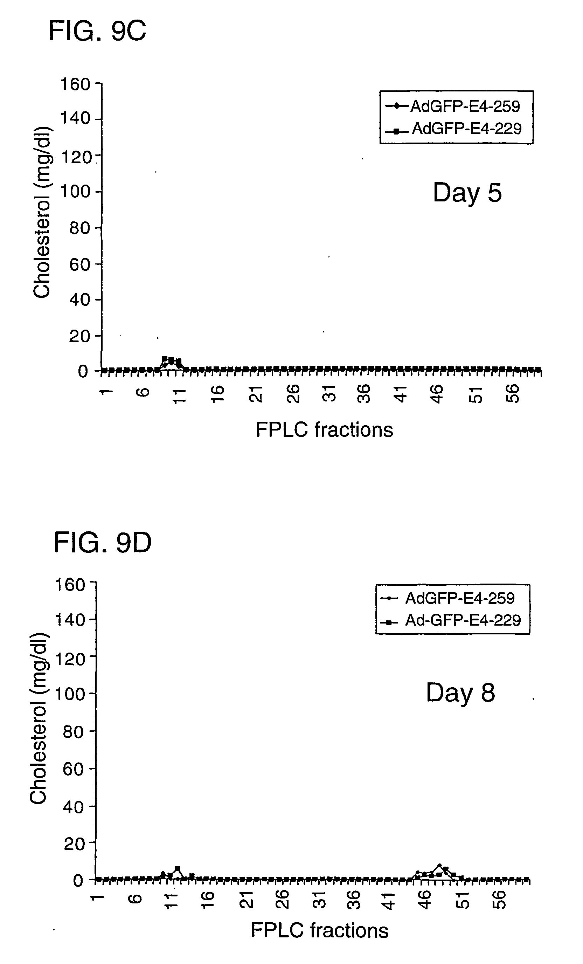

Figs. 9A-9D are graphs showing the cholesterol FPLC lipoprotein profiles of serum samples from apoE-deficient mice infected with 2x10⁹ pfu of AdGFP-E4 or 4x10⁹ pfu of AdGFP control virus (Figs. 9A and 9B) or with 4x10⁹ pfu of AdGFP-E4-229 or AdGFP-E4-259 (Figs. 9C and 9D). On days five and eight after infection, serum samples were collected and fractionated by FPLC on a Sepharose 6 column. Fractions were then analyzed for cholesterol content.

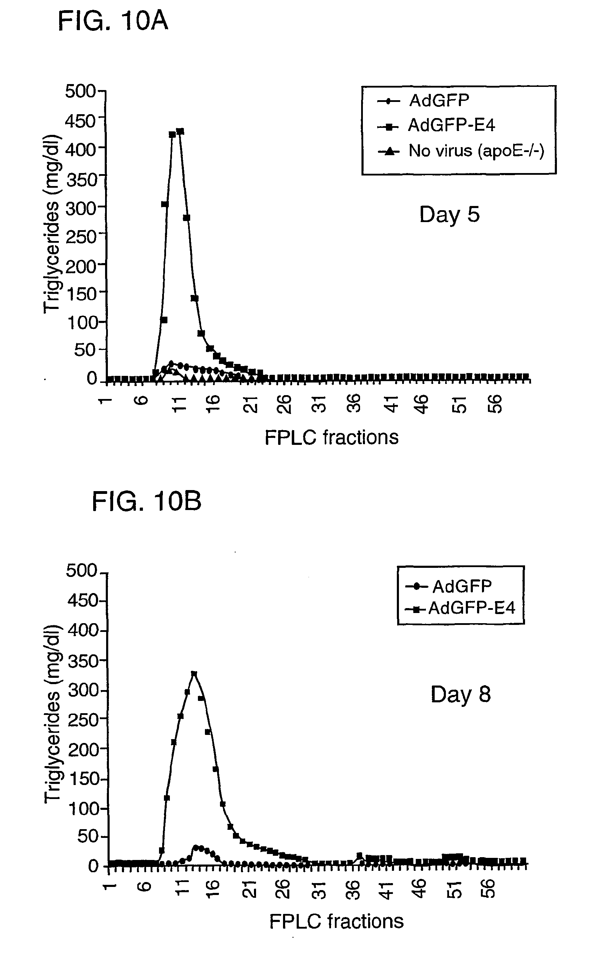

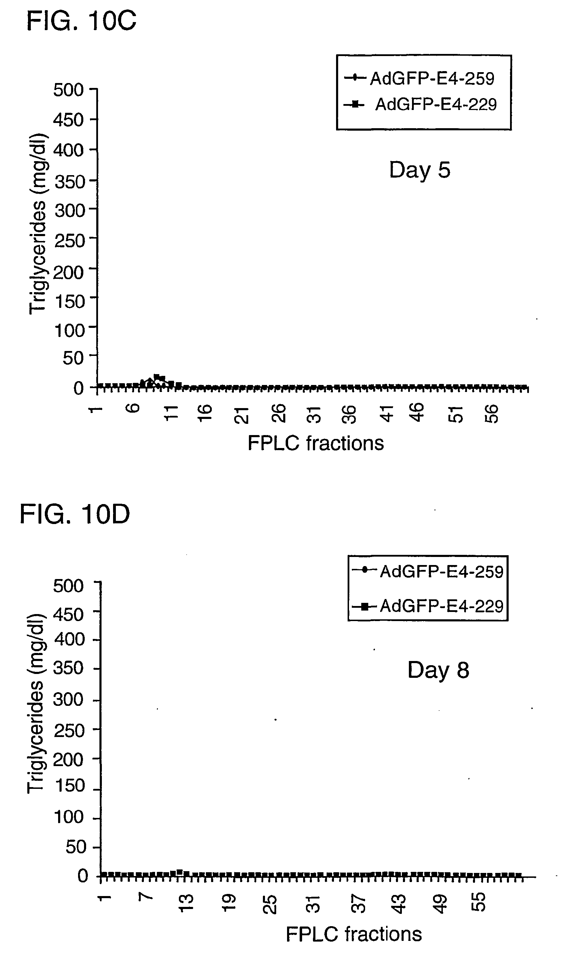

Figs. 10A-10D are graphs showing triglyceride FPLC lipoprotein profiles of serum samples from apoE-deficient mice infected with 2x10⁹ pfu of AdGFP-E4 or 4x10⁹ pfu of AdGFP control virus (Fig. 10A and 10B) or with 4x10⁹ pfu of AdGFP-E4-229 or AdGFP-E4-259 (Fig. 10C and 10D). On days five and eight after infection, serum samples were collected and fractionated by FPLC on a Sepharose 6 column. Fractions were then analyzed for triglyceride content.

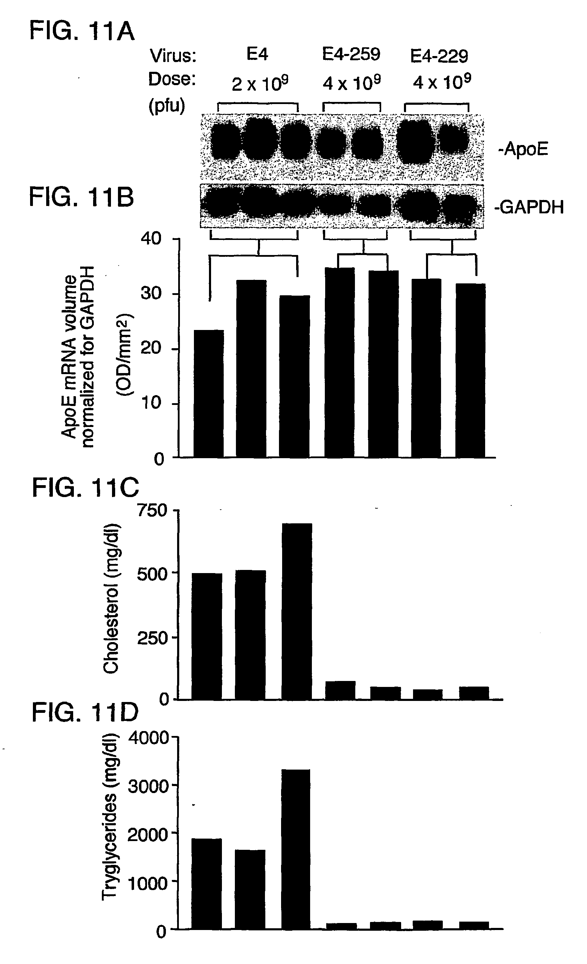

Figs. 11A and 11B are a Northern blot and a bar graph showing the *in vivo* mRNA expression of wt-apoE4, apoE4-229 and apoE4-259. ApoE-deficient mice infected with the indicated doses of the AdGFP-E4, AdGFP-E4-229 or AdGFP-E4-259 viruses were sacrificed five days after infection, and liver samples were collected. Then total RNA from the liver samples was isolated and analyzed by Northern blot analysis for the expression of apoE and GAPDH; a representative autoradiogram is shown in Fig. 11A. The apoE mRNA levels, normalized for GAPDH mRNA levels are graphed in Fig. 11B, showing that all three apoE mRNAs are expressed to similar levels. ApoE4 causes hypertriglyceridemia and fails to clear cholesterol; in contrast, apoE4-229 and apoE4-259 drastically reduce cholesterol without the unwanted side-effect of hypertriglyceridemia (Figs. 11C and 11D).

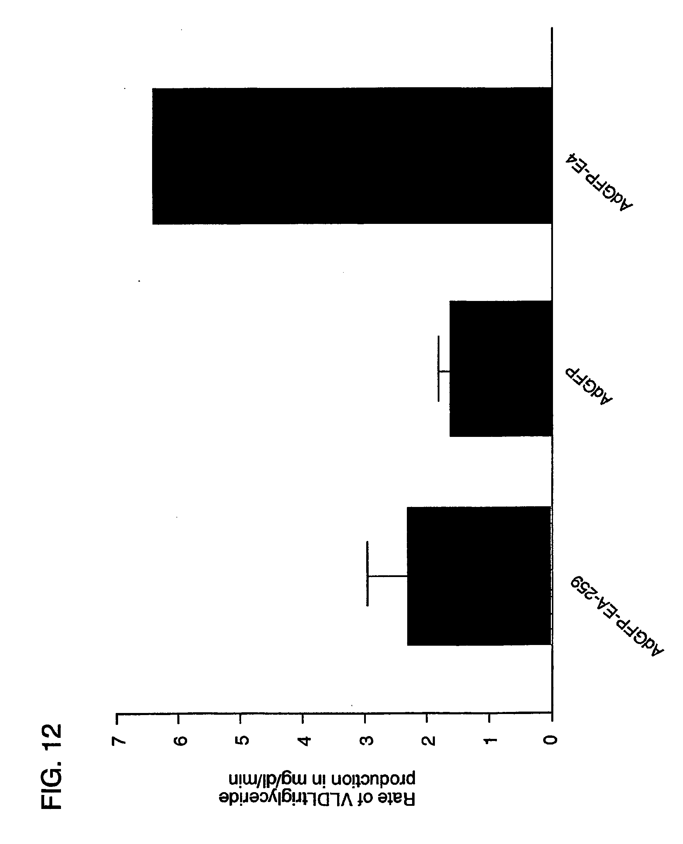

Fig. 12 is a bar graph of the average rate of VLDL triglyceride production *in vivo* for apoE-deficieat mice infected with AdGFP-E4-259, AdGFP-E4, or control AdGFP virus. Wild-type apoE4 induces a dramatic increase in VLDL triglyceride production compared to that induced by apoE4-259 or control virus. This failure of apoE4-259 to induce VLDL triglyceride production may contribute to the inability of the truncated apoE mutants to trigger hypertriglyceridemia.

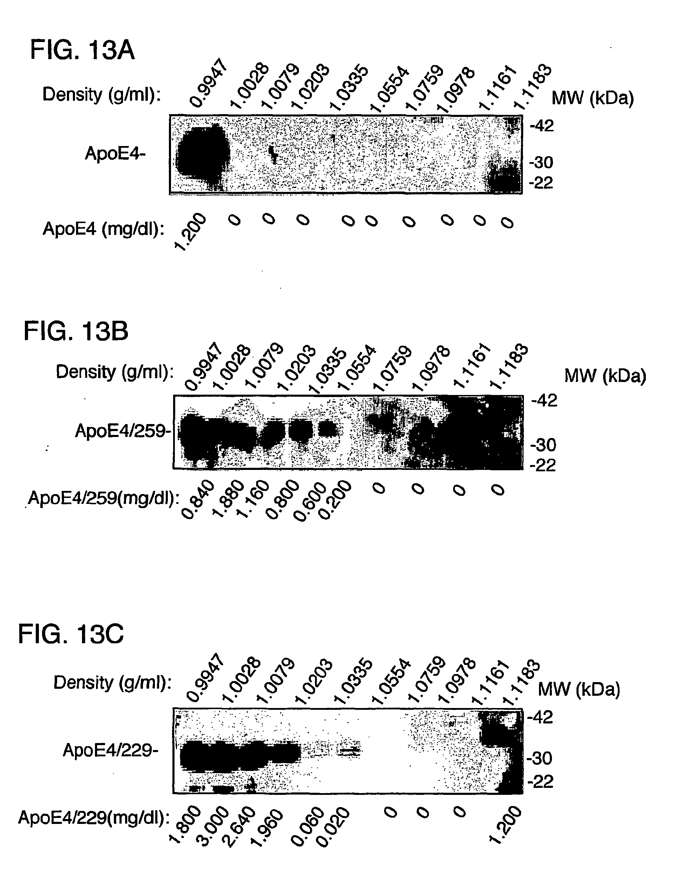

Figs. 13A-13C are pictures of gels showing that apoE4-229 and apoE4-259 can associate with smaller (higher density) apoE-deficient VLDL particles in addition to associating with the large (lowest density) apoE-deficient VLDL particles. The numbers below each lane of the gels represent the amount of apoE4 present in each lane in mg/dl, as determined by ELISA. The degree of association of apoE to apoE-deficient VLDL is similar for wild-type apoE4 and the apoE4-229 and apoE-259 truncated forms. The truncated forms of apoE4 have a greater association than wild-type apoE for higher density VLDL particles, which are smaller in size and have a lower triglyceride composition.

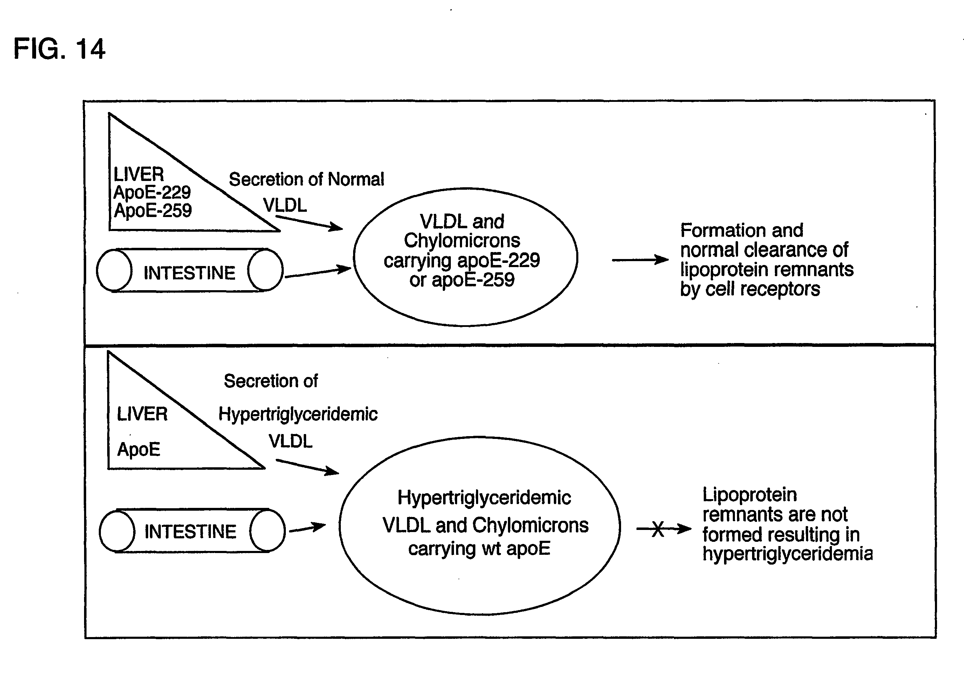

Fig. 14 is a schematic diagram of a model of the effects of overexpression of wild-type apoE, apoE-229, or apoE-259 on VLDL and chylomicron catabolism *in vivo.*

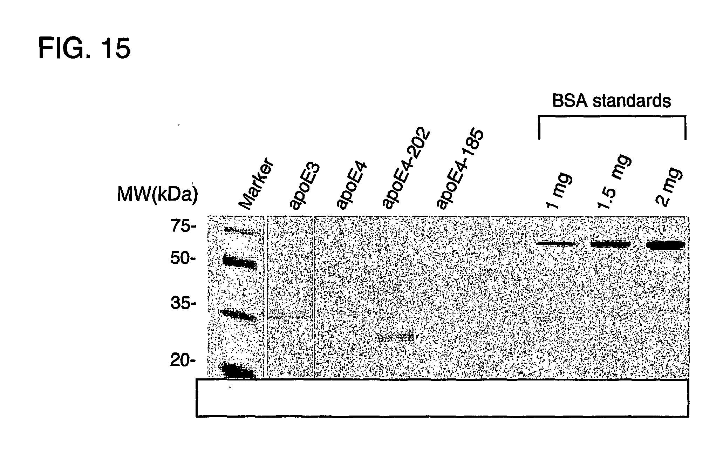

Fig. 15 is a picture of the SDS-PAGE analysis of the culture medium of HTB-13 cells infected with adenoviruses expressing apoE3, apoE4, apoE4-202 or apoE4-185. Fifteen µl of culture medium were analyzed. "Marker" indicates protein markers of different molecular weights.

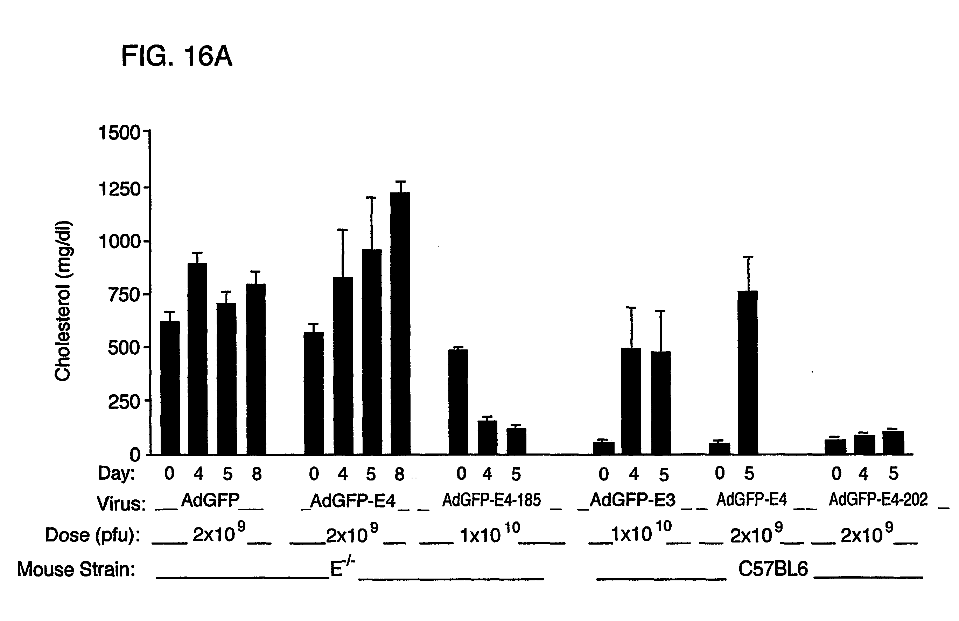

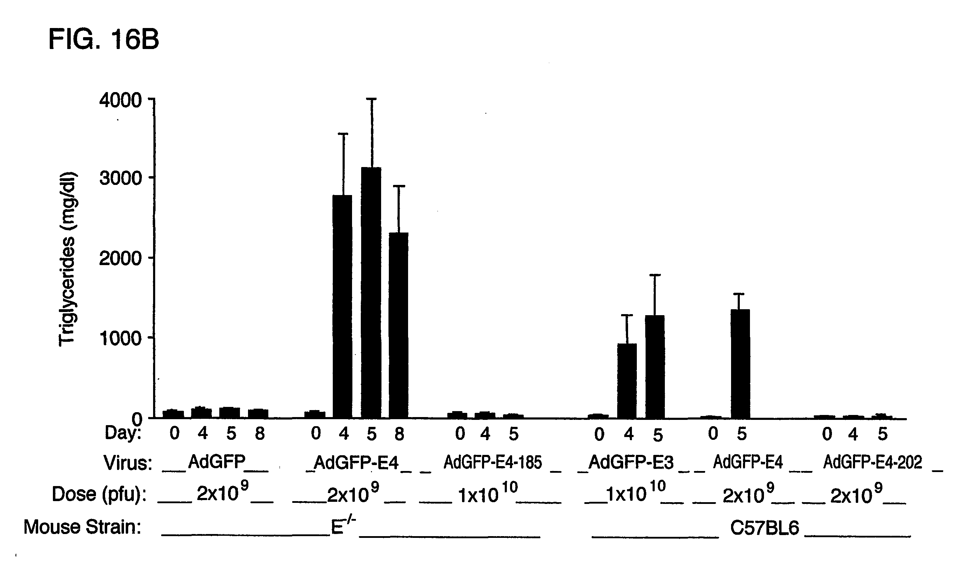

Figs. 16A and 16B are bar graphs of the cholesterol and triglyceride levels, respectively of apoE-deficient and C57BL6 mice infected with either the control adenovirus AdGFP, a recombinant adenoviruses expressing wild-type apoE, or a recombinant adenoviruses expressing a truncated apoE. Mice were infected in triplicate with the indicated doses of the indicated recombinant adenovirus, and serum samples were isolated and analyzed for cholesterol and triglyceride levels on the indicated days after infection as described herein.

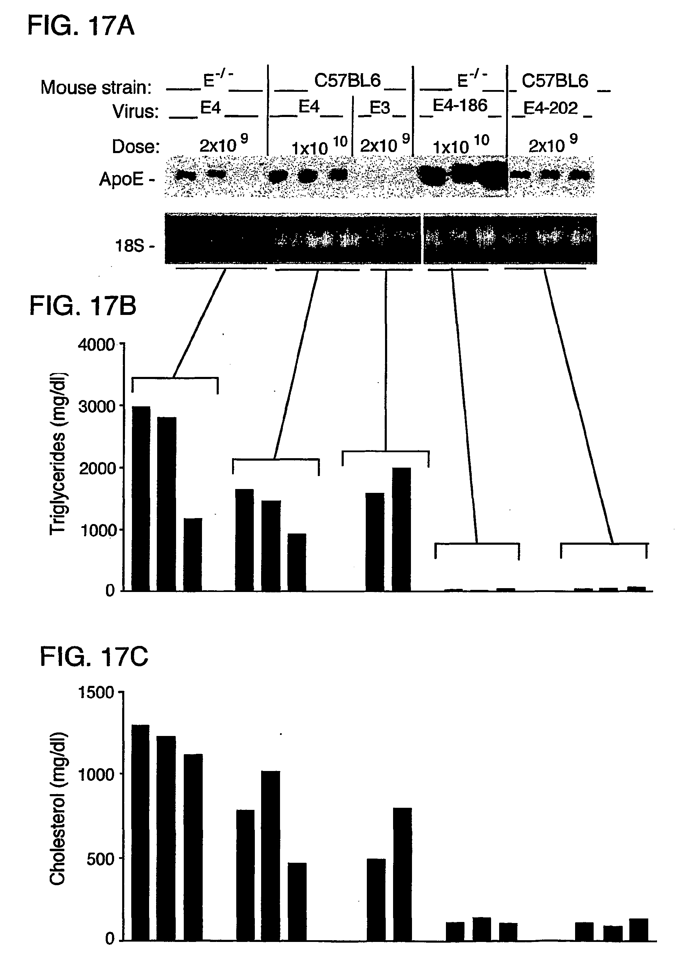

Fig. 17A is a picture of a representative autoradiogram of Northern blot analysis of mice infected with the indicated dose of the control adenoviruses AdGFP, a recombinant adenoviruses expressing wild-type apoE, or a recombinant adenoviruses expressing a truncated apoE. Total RNA was isolated from the livers of the infected mice on the indicated days after infection and analyzed by Northern blotting for the expression of apoE mRNA. Fig. 17A also shows the ethidium bromide staining of the gel for 18S ribosomal RNA as a control of RNA loading an integrity. Fig. 17B is a bar graph showing triglyceride levels of the individual mice expressed in mg/dl. Fig. 17C is a bar graph showing cholesterol levels of the individual mice expressed in mg/dl.

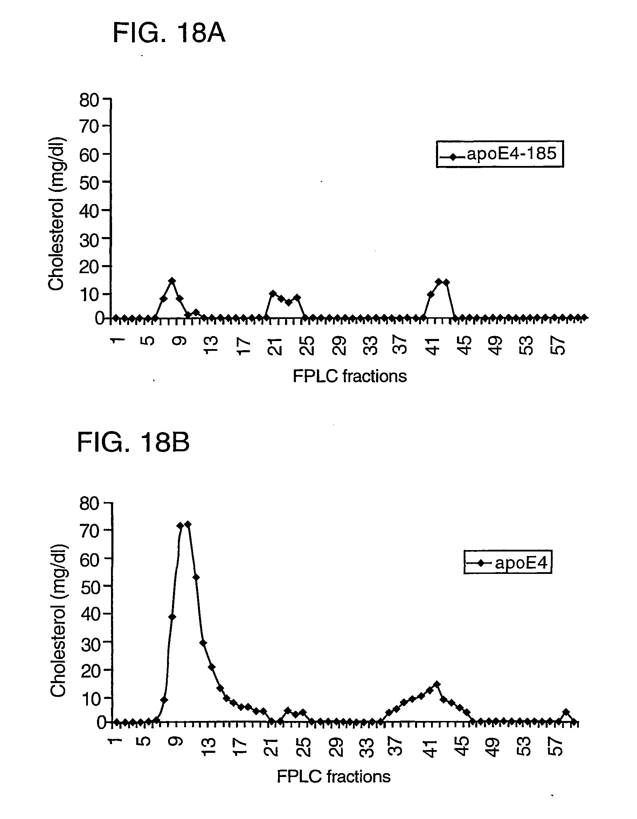

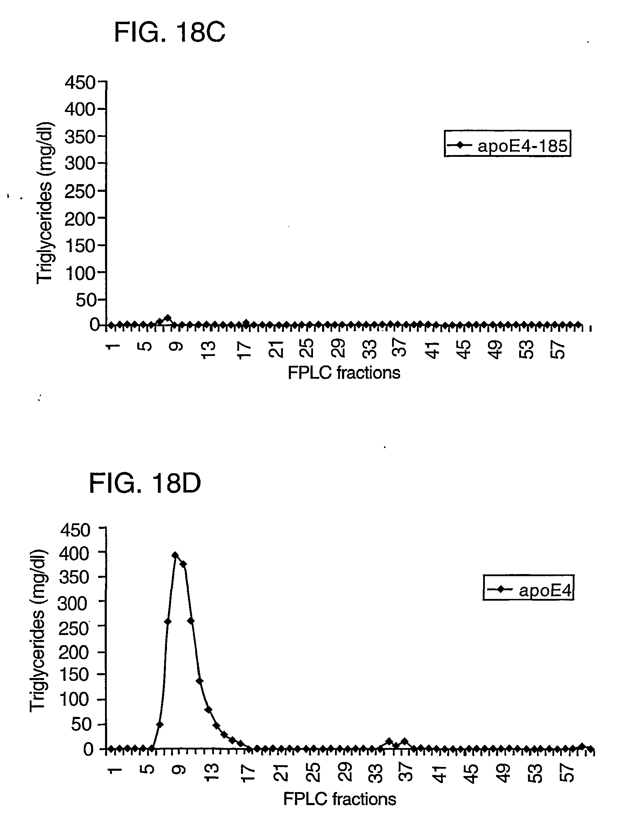

Figs. 18A-18D are graphs of the FPLC profiles of cholesterol and triglycerides of mice infected with the apoE4-185 (Figs. 18A and 18C, respectively) or apoE4 (Figs. 18B and 18D, respectively) expressing adenovirus. Five days after infection of mice with either 2x10⁹ pfu of the recombinant adenovirus expressing AdGFP-E4 or 1 x 10¹⁰ pfu of the recombinant adenovirus expressing AdGFP-E4-185, serum samples were obtained. These serum samples were fractionated by FPLC, and the cholesterol and triglyceride levels of each FPLC fraction were determined as described herein.

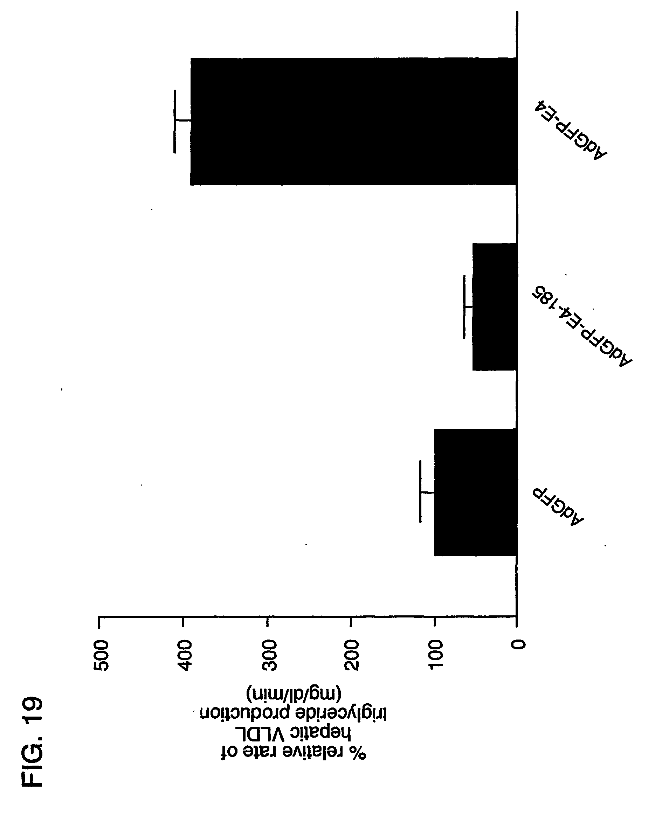

Fig. 19 is a bar graph of the average rate of hepatic VLDL-triglyceride production analysis in mice infected with AdGFP, AdGFP-E4, or AdGFP-E4-185. The bar-graph represents the mean +/- standard deviation of the individual rates of VLDL-triglyceride production per virus group.

### Detailed Description

Based on the hypothesis that distinct or overlapping regions of apoE mediate its role in cholesterol and triglyceride homeostatis, we used adenovirus-mediated gene transfer in apoE-deficient mice to dissect the domains of apoE required for cholesterol and triglyceride clearance *in vivo.* We previously reported that the amino-terminal 1-202 region of apoE contains the domains required for the association of apoE with lipoproteins and their subsequent clearance via cell receptors *in vivo.* Furthermore, the carboxy-terminal 203-299 residues of apoE contribute to apoE-induced hypertriglyceridemia *in vivo*. Additionally, we recently found that fragments of apoE containing the amino-terminal 1-185, 1-229, or 1-259 amino acids also significantly reduce cholesterol without inducing hypertriglyceridemia. Thus, removal of the carboxy-terminal 186-299, 230-299, or 260-299 residues of apoE also prevents apoE-induced hypertriglyceridemia *in vivo.*

Infection of apoE-deficient mice with an adenovirus expressing wild-type apoE4, dose of 2x10⁹ pfu, resulted in no significant clearance of cholesterol containing lipoproteins. In contrast, administering 2x10⁹, or even 1x10¹⁰ pfu, of a truncated version of apoE4 containing the amino-terminal 202 residues of apoE4 (apoE4-202) resulted in a 90% reduction in the cholesterol levels of apoE-deficient mice, without causing any significant increase in plasma triglyceride levels. Northern blot analysis of total RNA from the livers of the infected mice showed comparable levels of apoE4 and apoE4-202 mRNA. The findings suggest that the amino-terminal 1-202 region of apoE4 contains the domains required for the association of apoE with lipoproteins and their subsequent clearance via apoE-recognizing receptors *in vivo.* Furthermore, the carboxy-terminal 203-299 residues of apoE contribute to the apoE-induced hypertriglyceridemia *in vivo.* In the present study the clearance of lipoprotein remnants by apoE4-202, which contains the 142-147 heparin binding domain, but not the 211-218 or 243-272 heparin binding domains, is extremely efficient.

Northern blot analysis of total RNA established that, under conditions of similar steady-state apoE mRNA levels, mice expressing wild-type apoE/4 develop hypertriglyceridemia whereas those expressing apoE4-202 do not (Figs. 4A and 4B). This analysis indicates that it is unlikely that decreased apoE synthesis is responsible for this effect. Furthermore, tissue culture experiments showed that permanent cell lines expressing apoE4 or apoE4-202, or cells infected with recombinant adenovirus, secrete similar amounts of apoE4 and apoE4-202, indicating that the truncated apoE4-202 form is stable and is secreted as efficiently as the wild-type apoE4 counterpart.

Overexpression of full-length apoE3 or apoE4 in the liver of normal C57BL6 mice induced combined hyperlipidemia characterized by high plasma cholesterol and triglyceride levels. In contrast, overexpression of apoE4-202 did not induce high triglyceride levels. This result indicates that the induction of combined hyperlipidemia is the result of overexpression of apoE and is independent of the apoE phenotype. Additionally, induction of combined hyperlipidemia requires the carboxy-terminal region of apoE4 (amino acids 203 to 299).

X-ray crystallography and computer modeling show that the amino-terminal domains of apoE contain antiparallel helices (Wilson et al., Structure 2:713-718, 1994; Wilson et al., Science 252:1817-1822, 1991; Shimano, *supra;* Salah, *supra).* We believe that these amino-terminal helices extending from residues 23 to 155 may bind with specific architecture to the lipoprotein surface along with other protein particles. It is possible that at a critical apoE concentration, the apoE sites will be saturated and the clearance of apoE-containing lipoproteins will be optimized. With a further increase in plasma apoE concentration, specific displacement of other critical protein components of the triglyceride-rich lipoproteins may take place through the carboxy-terminal 203-299 region of apoE. This in turn may reduce the rate of lipolysis of these particles and increase plasma triglyceride levels (Figs. 7B). Thus, the lack of a significant increase in triglyceride levels after infection with apoE4-202 could be due to the greatly reduced ability of apoE4-202 to displace proteins from the VLDL particles. For example, some of the proteins present in triglyceride-rich VLDL (e.g., apoA-IV and apoA-I) are displaced by full-length apoE4 but not by apoE-202 (Fig. 7A). Additionally, it is possible that apoE4-202 decreases the secretion of triglyceride rich lipoproteins. However, the lack of this region (203-299) does not significantly impair the ability of the truncated protein to efficiently clear lipoprotein remnants.

Similar to the results seen for apoE4-202, administration of 4x10⁹ pfu of the adenovirus expressing apoE4-229 or apoE4-259 or 1x10¹⁰ pfu of the adenovirus expressing apoE4-185 to apoE-deficient mice significantly reduced serum cholesterol levels without increasing the level of serum triglycerides (Figs. 8A, 8B, 9A-9D, 10A-10D, 11C, 11D, 16A, and 16B). Northern blot analysis demonstrated that apoE4-229 and apoE4-259 mRNA were expressed at similar levels as wild-type apoE4 mRNA, suggesting that the failure of apoE4-229 and apoE4-259 to induce hypertriglyceridemia was not due to lower levels of expression of these apoE forms (Figs. 11A and 11B). Additionally, the level of apoE-185 mRNA in the liver of apoE-deficient mice infected with 1 x 10¹⁰ pfu of the adenovirus expressing apoE4-185 was higher than the level of apoE4 mRNA in mice infected with 2x10⁹ pfu of the adenovirus expressing apoE4. The rate of VLDL triglyceride production was much lower for apoE-259 than wild-type apoE, suggesting that this decrease in VLDL triglyceride production may contribute to the inability of the apoE truncated mutants to induce hypertriglyceridemia (Fig. 12). These results indicate that the carboxy-terminal 260-299 residues of apoE may be required for apoE-induced hypertriglyceridemia *in vivo.* Similarly, five days after infection, the rate of hepatic VLDL-triglyceride secretion in apoE-/- mice infected with the adenovirus expressing apoE4 was eight-fold higher than the rate of hepatic VLDL-triglyceride secretion in mice infected with the adenovirus expressing apoE4-185. The rate of VLDL-triglyceride secretion in mice infected with the truncated apoE4-185 form was even 50% lower than the corresponding rate in mice infected with the control AdGFP adenovirus.

While not meant to limit the invention to a particular theory, a model is proposed to account for the formation and normal catabolism of chylomicrons and VLDL in mice expressing the truncated apoE forms apoE4-185, apoE4-202, apoE4-229, or apoE4-259 and the defective clearance of triglyceride rich lipoproteins in mice overexpressing the full length apoE4 (Fig. 14). The model shows that overexpression of apoE is associated with formation of triglyceride-rich lipoproteins that cannot be cleared by cell receptors. In contrast, normal chylomicrons and VLDL particles formed in mice overexpressing apoE4-185, apoE4-202, apoE4-229, or apoE4-259 are removed efficiently by cell receptors, resulting in low plasma cholesterol and triglyceride levels in these mice.

The potential participation of the LRP and the LDL receptor in the clearance of the truncated apoE4-containing remnants may make the uptake of VLDL and the subsequent cholesterol clearance more efficient and thus account for the observed properties of truncated apoE4 in lipoprotein clearance. The efficient removal of lipoprotein remnants also removes concomitantly apoE molecules, leading to lower levels of steady state plasma apoE levels. The steady-state plasma apoE levels of the full-length apoE observed in this study are in the range of 60-70 mg/dl and the steady-state levels of the truncated apoE forms are in the range of 1 to 5 mg/dl.

Alternatively, the lipoproteins may be removed through the heparin sulfate proteoglycan pathway instead of, or in addition to, the LDL receptor and LRP pathways. Because the 142-147 heparin binding region in the truncated apoE4 proteins also contains the receptor binding domain, binding of apoE to heparin sulfate proteoglycans may mask the receptor binding domain and prevent recognition by cell-receptors. The resulting heparin sulfate proteoglycan-bound remnants may be cleared by direct endocytosis.

### Construction of Recombinant Adenoviruses Expressing apoE4 and Truncated Forms of apoE4

pUC-apoE4-202 was generated by PCR-mediated mutagenesis of codon 203 (GTA) to a stop codon (TGA) using pUC-apoE4 that was described previously (Aleshkov et al., Biochemistry 36:10571-10580, 1997) as a template and four sets of the oligonucleotides indicated in Table I, as primers. The set of external primers OUTPR1-Sense and OUTPR2-Antisense correspond to nucleotides encoding amino acids 103-111 and 208-215 of apoE respectively, and contain the restriction sites NgoMI and BstEII, respectively. The set of mutagenic oligonucleotides extending 9 residues 5' and 9 residues 3' of codon 203 has been altered in its sequence to a stop codon (INTERNAL-Sense-203 and INTERNAL-antisense-203, Table I).

**Table I. Oligonucleotides used in overlap extension PCR**

| | |
|---|---|
| | |

| Oligo Name | Oligo Sequence |
|---|---|
| OUTPR1-Sense | 5'-GCT GGG TGC AGA CAC TGT CTG AGC-3' |
| OUTPR2-Antisense | 5'-CGC AGC CGC TCG CCC CAG CAG GCC T-3' |
| INTERNAL-Sense-186 | 5'-CCC CTG GTG **TAA** CAG GGC CGC GTG-3' |
| INTERNAL-Antisense-186 | 5'-GCG GCC CTG **TTA** CAC CAG GGG CCC-3' |
| INTERNAL-Sense-203 | 5'-GGC CAG CCG ***TGA*** CAG GAG CGG-3' |
| INTERNAL-Antisense-203 | 5'-CCG CTC CTG ***TCA*** CGG CTG GCC-3' |
| INTERNAL-Sense-230 | 5'-C GAC CGC CTG ***TAA*** GAG GTG AAG G-3' |
| INTERNAL-Antisense-230 | 5'-C CTT CAC CTC ***TTA*** CTG GTG AAG G-3' |
| INTERNAL-Sense-260 | 5'-A TTC CAG GCC ***TAA*** CTC AAG AGC T-3' |
| INTERNAL-Antisense-260 | 5'-A GCT CTT GAG ***TTA*** GGC CTG GAA T-3' |

| | |
|---|---|
| **Bold underlined* bases represent the mutated codon. | |

The PCR-based mutagenesis of codon 203 involved two separate amplification reactions. The first reactions used the 5' external primer and the antisense mutagenic primer covering codon 203. The second reaction used the 3' external primer and the sense mutagenic primer covering codon 203. An aliquot of 4% of the volume of each PCR reaction was mixed, and the sample was amplified by the 5' and the 3' external primers. The amplified fragment was then digested with NgoMI and BstEII and used to replace the wild-type sequence of the pUC-E4 plasmid. pUC-apoE4-185, pUC-apoE4-229, and pUC-apoE4-259 were constructed similarly using the corresponding mutagenic primers to covert codon 186, 230, or 260 to a stop codon, respectively.

To incorporate the PCR-generated mutations to exon IV of the apoE gene, a two-step procedure was followed. The EcoRI fragment of the human apoE4 gene, which includes the entire exon IV sequence, was cloned into the EcoRI site of the pBS vector to generate the vector pBlue-E4-exIV. The vector pBlue-E3-exIV was constructed similarly using a EcoRI fragment of the human apoE3 gene. The pUC-apoE4-185, pUC-apoE4-202, pUC-apoE4-229, and pUC-apoE4-259 plasmids were digested with StyI/BbsI and the mutated sequence was exchanged for the wild-type sequence of the pBlue-E4-exIV plasmid to generate plasmids pBlue-E4-185-exIV, pBlue-E4-202-exIV, pBlue-E4-229-exIV, and pBlue-E4-259-exIV (Fig. 1).

The recombinant viruses were constructed using the Ad-Easy-1 system where the recombinant adenovirus construct is generated in bacteria BJ-5183 cells (He et al., Proc. Natl. Acad. Sci. USA 95:2509-2514, 1998). The 1507 bp MscI-EcoRI fragment of apoE genomic DNA (nucleotides 1853 to 3360) which contains exons 2 and 3, was cloned into the SmaI-EcoRI sites of pGEM7 vector, resulting in the pGEM7 apoE-Ex II-III vector. The 1,911 bp EcoRI fragment of apoE3, apoE4, apoE4-185 (which contains the stop mutation at codon 186), apoE4-202 (which contains the stop mutation at codon 203), apoE4-229 (which contains the stop mutation at codon 230), and apoE4-259 (which contains the stop mutation at codon 260) was then excised from the pBlue-E3-Ex IV, pBlue-E4-Ex IV, pBlue-E4-185-Ex IV, pBlue-E4-202-Ex IV, pBlue-E4-229-Ex IV, and pBlue-E4-259-Ex IV vectors, respectively, and cloned into the EcoRI site of the pGEM7- ExII,III vector. This generated the pGEM7-apoE3g, pGEM7-apoE4g, pGEM7-apoE4g-185, pGEM7-apoE4g-202, pGEM7-apoE4g-229, and pGEM7-apoE4g-259 vectors respectively, that contain exons II, III, and IV of the apoE gene. The correct orientation of the 1,911 bp EcoRI insert was checked by restriction digestion with NotI and XbaI. The entire HindIII-XbaI fragment from the pGEM7-apoE3g, pGEM7-apoE4g, pGEM7-apoE4g-185, pGEM7-apoE4g-202, pGEM7-apoE4g-229, and pGEM7-apoE4g-259 vectors was cloned into the corresponding sites of the pAd Track-CMV adenovirus shuttle plasmid.

Each recombinant vector was used to electroporate BJ 5183 *E. coli* cells along with the pAd-Easy-1 helper vector. pAdEasy-1 contains the viral genome and the long terminal repeats of the adenovirus and allows for the formation by homologous recombination of the recombinant virus containing the gene of interest. The vector also contains the green fluorescence protein gene which enables detection of the infection of cells and tissues by their green fluorescence. Recombinant bacterial clones resistant to kanamycin were selected and screened for the presence of the gene of interest by restriction endonuclease analysis and DNA sequencing. The viruses expressing wild-type apoE3, apoE4, apoE4-185, apoE4-202, apoE4-229, or apoE4-259 forms are designated as AdGFP-E3, AdGFP-E4, AdGFP-E4-185, AdGFP-E4-202, AdGFP-E4-229, or AdGFP-E4-259, respectively. Correct clones were propagated in RecA-deficient DH5α cells. The recombinant vector was linearized with PacI and used to infect 911 cells. The subsequent steps involved in the generation and expansion of recombinant adenoviruses were plaque identification/isolation followed by infection and expansion in 911 cells (van Dijk, *supra).* These steps were followed by a purification process involving CsCl ultracentrifugation performed twice, followed by dialysis and titration of the virus. Usually, titers of approximately 5x10¹⁰ pfu/ml were obtained.

### Cell Culture Studies

Human HTB13 cells (SW1783, human astrocytoma) grown to confluence in medium containing 10% fetal calf serum, were infected with AdGFP-E4, AdGFP-E4-202, AdGFP-E4-229, or AdGFP-E4-259, at a s multiplicity of infection of 20. Twenty-four hours post-infection, cells were washed twice with phosphate buffered saline (PBS), and preincubated in serum free medium for two hours. Following an additional wash with PBS, fresh serum free medium was added. After a 24 hours incubation, medium was collected and analyzed by enzyme linked immunoabsorbent assay (ELISA) and SDS-PAGE for apoE expression. In some experiments, 60 mm diameter cultures were labeled metabolically with 0.5 *µ*Ci of ³⁵S methionine for 2 hours, in which case medium was collected 2 hours after addition of the radiolabeled amino acid for further analysis.

### Separation of the ApoE-Containing Lipoproteins Secreted into the Culture Medium of Cells

After the labeling of cells with ³⁵S methionine, 2 ml medium from one 100 mm diameter dish was adjusted to density of 1.35 g/ml with KBr and overlayed with 2 ml each of KBr solutions of density =1.25, 1.115, 1.06 g/ml and 2 ml of normal saline. The mixture was centrifuged for 24 hours in SW-41 rotor at 30,000 rpm. In some tubes the culture medium was mixed with 0.8 ml lipoprotein fractions with a density of 1.006-1.21 g/ml, which were previously separated from plasma by density gradient ultracentrifugation. Following ultracentrifugation, twelve 1 ml fractions were collected and analyzed by SDS-PAGE and autoradiography. This analysis showed that both apoE4 and apoE4-202 can associate with exogenous lipoproteins which float in the IDL to HDL region.

### Animal Studies in apoE-Deficient Mice

Female apoE-deficient mice (van Ree et al., Atherosclerosis 111:25, 1994) 20-25 weeks old were used in these studies. Groups of mice were formed based on their plasma cholesterol and triglyceride levels before initiation of the experiments, to ensure similar mean cholesterol and triglyceride levels in each group. The mice were injected intravenously through the tail-vein with doses ranging from 5x10⁸ to 1x10¹⁰ pfu of AdGFP (control adenovirus), AdGFP-E4 or AdGFP-E4-202 virus or with 4x10⁹ pfu of AdGFP-E4-229 or ADGFP-E4-259. Each group contained 8-10 mice. Blood was obtained from the tail vein or retro orbital plexus after a 4 hour fast preceding adenoviral injection. On the indicated days after injection, blood was collected into a CB300 or CB1000 blood-collection tube (Sarstedt). Aliquots of plasma were stored at 4° and -20°C. One or more animals from each group was sacrificed on each of the indicated days such that mRNA expression in the mouse liver could be analyzed.

### FPLC Analysis of Serum Samples

For FPLC analysis of serum samples from AdGFP, AdGFP-E4, AdGFP-E4-185, or AdGFP-E4-202 treated mice, 12 *µ*l of serum were diluted 1:5 with PBS. Then the sample was loaded onto a Sepharose 6 column in a SMART micro FPLC system (Pharmacia), and eluted with PBS. A total of 25 fractions of 50 *µ*l volume each were collected for further analysis. For samples from AdGFP-E4-229 or ADGFP-E4-259 treated mice, 100 µl of serum was diluted 1:1 with PBS, and the analysis was performed similarly using a regular FPLC system instead of a micro FPLC system. A total of 60 fractions of 250 µl each were collected.

### Triglyceride and Cholesterol Analysis

Ten µl of serum sample were diluted with 40 µl phosphate buffered saline (PBS), and 7.5 µl of the diluted sample were analyzed for triglycerides and cholesterol using the GPO-Trinder Kit (Sigma) and CHOL-MPR3 kit (Boehringer-Mannheim), according to the manufacturers instructions. Triglyceride and cholesterol concentrations were determined spectrophotometrically at 540 nm and 492 nm, respectively. Triglyceride analysis of the FPLC fractions was performed using the TG-Buffer (Sigma), and concentrations were determined spectrophotometrically at 492 nm according to the manufacturer's instructions. Cholesterol analysis of the FPLC fractions was performed as described above.

### Quantification of Human ApoE Protein Levels

Serum human apoE4 concentrations were measured by using sandwich ELISA (Kim et al., J. Biol. Chem. 271:8373-8380,1996). Affinity purified polyclonal goat anti-human apoE antibodies were used for coating and polyclonal goat anti-human apoE conjugated to horseradish peroxidase was used as the secondary antibody. After incubation of the plates with the goat anti-human apoE conjugated to horseradish peroxidase, detection was performed using the immunoperoxidase procedure with tetramethylbenzidine as the substrate. Pooled plasma from healthy human subjects with known apoE level was used as a standard.

### Isolation of VLDL by Density Gradient Ultracentrifugation

500 *µ*l of serum sample obtained from adenovirus-infected mice and apoE-deficient mice were overlaid on a KBr solution composed of 2 ml of 1.21 g/ml KBr, 2.5 ml 1.063 g/ml KBr, 2.5 ml of 1.019g/ml KBr, and 2 ml of ddH₂O. Samples were subjected to ultracentrifugation at 40,000 rpm for 16 hours, and then the top 1 ml of the gradient containing the VLDL fraction, was isolated

### ApoE Enrichment of VLDL Particles

Five hundred microliters of VLDL, isolated from the plasma of apoE-deficient mice, was mixed with culture medium containing 250 µg of either apoE4, apoE4-202, apoE4-229, or apoE4-259 secreted by HTB cells, infected with AdGFP-E4, AdGFP-E4-202, AdGFP-E4-229, or AdGFP-E4-259, respectively, to a final volume of 1 ml. Mixtures were incubated on a shaker at 37°C for 1 hour, and then subjected to density gradient centrifugation to separate free apoE from the VLDL-bound apoE. Then, the apoE-enriched VLDL, and free apoE fractions were isolated and analyzed for apoE concentration by SDS-PAGE and ELISA.

### SDS-Polyacrylamide Gel Electrophoresis and Western Blot Analysis

From each lipoprotein fraction, samples of 7.5 µg of protein were analyzed for apolipoproteins by SDS-polyacrylamide gel electrophoresis (SDS-PAGE) with 12% gels. Proteins were detected by silver staining or Coomassie brilliant blue staining.

### RNA Isolation and Hybridization Analysis

Total RNA was isolated from liver samples of the infected mice using the RNA Easy solution (RNA Insta-Pure, Eurogentec Belgium) according to the manufacturer's instructions. For Northern blot analysis, RNA samples (15 *µ*g) were denatured and separated by electrophoresis on 1.0% formaldehyde-agarose gels. RNA was stained with ethidium bromide to verify integrity and equal loading and then transferred to GeneScreen Plus (DuPont NEN). RNA was cross-linked to the membrane by UV irradiation (Stratalinker, Stratagene) at 0.12 joules/cm² for 30 seconds. Probes, prepared by random priming, were used as described previously (Kypreos et al., J. Cell. Biochem. 68:247-258, 1998) with 2.0 x 10⁶ cpm/ml [³²P]-labeled DNA. Quantitation by scanning densitometry was performed using a Molecular Dynamics phosphorimager (Model 400B). ApoE mRNA expression was normalized for GAPDH mRNA levels, and reported in the form of a bar graph. Experiments were performed in triplicate, and data are reported as the mean value (Figs. 4A, 4B, 11A, and 11B).

### Association of ApoE4-202 with Lipoproteins Secreted by Adenovirus-Infected HTB-13 Cell Cultures

HTB-13 cells that do not synthesize endogenous apoE were infected with recombinant adenoviruses expressing apoE4 or apoE4-202, designated AdGFP-E4 and AdGFP-E4-202, respectively, at a multiplicity of infection of 20. Analysis of the culture medium by SDS-PAGE and sandwich ELISA showed that both apoE4 and apoE4-202 are secreted efficiently at comparable levels in the range of 60 to 80 µg of apoE per ml, 24 hours after infection with AdGFP-E4 or AdGFP-E4-202. Density gradient ultracentrifugation showed that although the majority of apoE was in the lipid-poor or lipid-free fraction, apoE4 and apoE4-202 can associate with exogenously added lipoproteins in the density = 1.04 to 1.21 g/ml fractions (Figs. 2A and 2B). The data indicate that the truncated apoE form apoE4-202 has the ability to associate with pre-existing lipoprotein particles, a process that is required for receptor-mediated lipoprotein clearance. To establish further the ability of apoE4 and apoE4-202 to associate with lipoprotein remnants, equal amounts of apoE4 and apoE4-202 were mixed with serum isolated from the plasma of apoE deficient mice, and incubated at 37°C for 1 hour. The mixture was then subjected to density gradient ultracentrifugation. The amounts of the free and lipoprotein-associated apoE were then assessed quantitatively by ELISA and qualitatively by fractionation on SDS-PAGE followed by Coomassie Brilliant blue staining of the gel and comparison of the intensity of the apoE band to bovine serum albumin (BSA) standards. As shown in Figs. 2C and 2D, both the full-length apoE4 and the truncated apoE4-202 associate with the VLDL.

### Similar Levels of Expression and Secretion of apoE4-185 by HTB-13 Cells

The amount of apoE4-185 secreted by HTB-13 cells was compared to the amount of apoE3, apoE4, and apoE-202 secreted by these cells. For this assay, HTB-13 cells were infected with recombinant adenoviruses expressing apoE3, apoE4, apoE4-202, or apoE4-185 at a multiplicity of infection of 20, as described above. Analysis of the culture medium by sandwich ELISA showed that the full length and truncated apoE forms are secreted into the culture medium at comparable levels in the range of approximately 60 to 70 *µ*g of apoE per ml, 24 hours after infection. This result is similar to the range of 60 to 80 *µ*g of apoE per ml obtained from the experiment described above for apoE4 and apoE4-202. A similar quantitative assessment was obtained by SDS-PAGE analysis of the secreted protein using known BSA standards (Fig. 15).

### Contribution of the Carboxy Terminal 186-299, 203-299, 230-299, and 260-299 Segments of ApoE to Hypertriglyceridemia in ApoE-Deficient Mice

To assess the effects of apoE4 and apoE4-202 on hyperlipidemia *in vivo*, apoE-deficient mice (apoE-/-) were infected with the recombinant adenoviruses AdGFP-E4 or AdGFP-E4-202 respectively. To assess potential non-specific effects of virus infection, some mice were infected with the control AdGFP virus. Analysis showed that the infection of mice with 2x10⁹ pfu of the apoE4-adenovirus did not result in a significant reduction in the mouse cholesterol levels compared to the control infection, and induced severe hypertriglyceridemia (Figs. 3A and 3B). Hypertriglyceridemia was the result of overexpression of the apoE4. When expression of apoE4 on day 8 post-infection was reduced or when the dose used for infection was decreased, hypertriglyceridemia was also reduced or eliminated (Fig. 3A). When mice were infected with 2x10⁹ or even 10¹⁰ pfu of the AdGFP-E4-202 adenovirus, hypertriglyceridemia was not induced (Fig. 3A). The control virus AdGFP did not appear to have significant effects on the cholesterol and triglyceride levels of the apoE-deficient mice, ruling out the possibility of non-specific effects of the infection process.

Similar to the results observed using apoE4-202, infection of apoE-deficient mice with 4x10⁹ pfu of AdGFP-E4-229 or AdGFP-E4-259 reduced cholesterol levels without inducing hypertriglyceridemia (Figs. 8A and 8B). Additionally, infection of apoE-deficient mice with 1x10¹⁰ pfu of apoE4-185-expressing virus normalized cholesterol levels without inducing hypertriglyceridemia (Figs. 16A and 16B). This finding indicates that the amino-terminal residues 1-185 of apoE contain all the determinants required for clearance of the lipoprotein remnants which accumulate in the plasma of the apoE-/- mice.

### Contribution of the Carboxy-Terminal 203-299 to Hypercholesterolemia, and Hypertriglyceridemia in Normal C57BL6 Mice

The hypertriglyceridemia induced in apoE-/- mice by overexpression of the human apoE4 could be the consequence of the underlying hypercholesterolemia in apoE-/- mice. Alternatively, full-length apoE could by itself elicit high plasma lipid levels. To differentiate between these two possibilities, normal C57BL6 mice (apoE+/+) were infected with 1 to 2 x 10⁹ pfu of adenoviruses expressing apoE3, apoE4, or apoE4-202. This analysis showed that overexpression of either apoE3 or apoE4 was sufficient to induce combined hyperlipidemia (high cholesterol and triglyceride levels) in normal C57BL6. In contrast, overexpression of apoE4-202 had no detectable effect on triglyceride levels of the C57BL6 mice (Figs. 16A and 16B). This results indicates that overexpression of full-length apoE is sufficient to cause combined hyperlipidemia in normal C57BL6 mice or apoE-/- mice. Furthermore, the induction of hyperlipidemia requires the carboxyterminal region of apoE. While not meant to limit the invention to a particular theory, the domains of apoE within the carboxyterminal 203-299 region may contribute directly to the secretion of lipoprotein particles which are not removed efficiently by the liver, resulting in high plasma cholesterol and triglyceride levels.

### Similar Expression of ApoE4, ApoE4-185, ApoE4-202, ApoE4-229, and ApoE4-259 mRNA In vivo

To assess the expression of apoE4, apoE4-202, apoE4-229, and apoE4-259 mRNA in apoE-deficient mice infected with AdGFP-E4, AdGFP-E4-202, AdGFP-E4-229, or AdGFP-E4-259, respectively, at least 3 infected mice from each group were sacrificed on day 5 post-infection, and their livers were collected (Figs. 8A and 8B). Total RNA was isolated from these livers and analyzed for apoE mRNA expression by Northern blot analysis. As shown in Figs. 4A, 4B, 11A, and 11B, the apoE mRNA levels in mice infected with a dose of 2x10⁹ pfu of AdGFP-E4 are similar to those in mice infected with 1x10¹⁰ pfu AdGFP-E4-202 or 4x10⁹ pfu of AdGFP-E4-229 or AdGFP-E4-259. However, apoE4-202, apoE4-229, and apoE4-259 do not cause hypertriglyceridemia while they effectively correct hypercholesterolemia (high blood cholesterol levels), as opposed to full-length apoE4. Thus, the different effects of apoE4 and apoE4-202, apoE4-229, or apoE4-259 on hypertriglyceridemia are not due to different levels of expression between these apoE forms.

Additionally, the steady-state apoE4-185 mRNA level in apoE-deficient mice infected with AdGFP-E4-185 was compared to the corresponding level of full-length apoE4 mRNA in mice infected with AdGFP-E4. For this comparison, at least 2-3 infected mice from each group were sacrificed on day 5 post-infection, and their livers were collected as described above. Total RNA was isolated from these livers and analyzed for apoE mRNA levels by Northern blotting. Ethidium bromide staining of the gel for 18S ribosomal RNA was used as an index of equal loading and integrity of the RNA (Fig. 17A). The apoE mRNA levels of apoE-/- mice infected with 1x10¹⁰ pfu of AdGFP-E4-185 are greater than the mRNA levels in mice effected with 2x10⁹ pfu of AdGFP-E4 (Fig. 17A). However, apoE4-185 does not cause hyperlipidemia, as opposed to full-length apoE4 which causes high cholesterol and triglyceride levels (Figs. 17B and 17C). Thus, the failure of apoE4-185 to induce hyperlipidemia is most likely not due to differences in the expression between these two apoE forms (Figs. 17A-C).

The mRNA levels of apoE3, apoE4, or apoE4-202 in normal C57B16 mice infected with the corresponding adenovirus were compared. In agreement with the cell culture data of Fig. 15, the apoE mRNA levels in C57BL6 mice infected with adenoviruses expressing apoE3, apoE4, or apoE4-202 (at a dose of 2 x10⁹ for apoE3 and apoE4-202 and at a dose of 1 x10¹⁰ pfu for apoE4) are comparable (Fig. 17A).

### Cholesterol, Triglyceride, and ApoE FPLC Profiles of Plasma Isolated from Mice Infected with AdGFP-E4, AdGFP-E4-202, AdGFP-E4-229, AdGFP-E4-259, or the Control Virus AdGFP

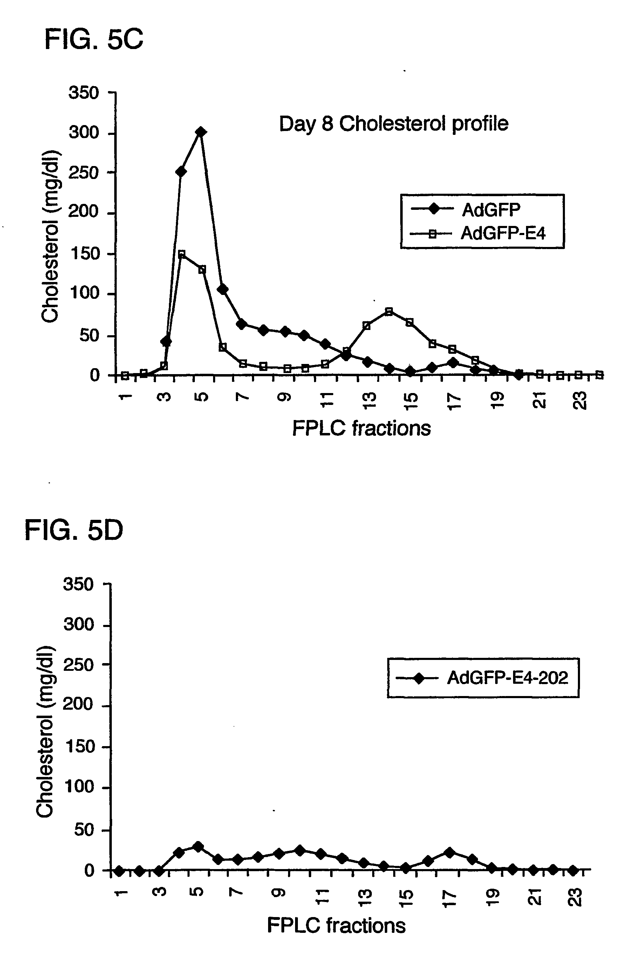

FPLC analysis of the plasma from adenovirus-infected apoE-deficient mice showed that in mice expressing apoE4, 72% of the cholesterol was distributed in the VLDL fractions and approximately 18% in the HDL fractions five days after infection (Fig. 5A). On day 8 post-infection, the ratio of VLDL/HDL cholesterol was approximately 1:1 in mice infected with AdGFP-E4 (Fig. 5C). In mice infected with AdGFP-E4-202, cholesterol was distributed in the VLDL and the HDL3 fractions and the ratio of VLDL cholesterol to HDL cholesterol was approximately 3:2 on days 5 and 8 post-infection, using either 2x10⁹ or 10¹⁰ pfu of adenovirus (Figs. 5B and 5D). As expected, infection with 2x10⁹ pfu of the control virus AdGFP, did not result in any change in the cholesterol and triglyceride profiles of the apoE-deficient mice (data not shown).

The FPLC analysis also showed that VLDL-triglyceride levels were elevated in mice on days 5 and 8 post-infection with AdGFP-E4 (Figs. 5E, 5F, 10A, and 10B). In contrast, VLDL-triglyceride levels were not elevated in mice infected with AdGFP-E4-202, AdGFP-E4-229, AdGFP-E4-259, or AdGFP (Figs. 5E, 5F, 10C, and 10D).

Analysis of FPLC fractions by sandwich ELISA showed that in mice infected with 2x10⁹ pfu AdGFP-E4, approximately 50% of the total apoE was distributed in the HDL fractions and 25% in the VLDL fractions, with the remaining apoE being distributed across the other FPLC fractions on day 5 post-infection (Fig. 6). In contrast, in mice infected with 10¹⁰ pfu AdGFP-E4-202, the protein was uniformly distributed in all lipoprotein fractions. The apparent lower concentration of apoE4-202 in the lipoprotein-containing fractions reflects the efficient catabolism of the apoE4-202-containing lipoproteins. The levels of apoE4 and apoE4-202 were similar in the FPLC fractions 22-25 containing the lipid-free apoE, suggesting that there are similar steady-state levels for lipid-free apoE4 and apoE4-202 in the plasma of mice infected with 2x10⁹ pfu AdGFP-E4 or 10¹⁰ pfu AdGFP-E4-202, respectively.

FPLC analysis of plasma from apoE-/- mice infected with AdGFP-E4-185 showed that cholesterol was present in low levels and distributed in the VLDL, LDL, and HDL at a ratio of approximately 2:1:1 (Fig. 18A). The triglyceride levels in these mice were low in the VLDL fraction and barely detectable in the rest of the lipoprotein fractions (Fig. 18C). In contrast, mice expressing apoE4 had high levels of cholesterol five days after infection. Approximately 80% of cholesterol was distributed in VLDL and approximately 20% in HDL (Fig. 18B). As expected, triglyceride levels were very high in the VLDL fractions and barely detectable in the rest of the lipoprotein fractions (Fig. 18D). As an additional control, infection with 2x10⁹ pfu of the control virus AdGFP did not result in any detectable change in the cholesterol and triglyceride profiles of the apoE-deficient mice.

The average level of total plasma apoE was measured by sandwich ELISA on a pool of plasma from three mice infected with the same adenovirus. The amount of plasma apoE protein was 60-70 µg/dl for apoE3 and apoE4 and 1-5 µg/dl for apoE4-202 and apoE4-185.

### Apoprotein Composition of VLDL in Mice Infected with Control and ApoE-Expressing Adenoviruses

Analysis of VLDL isolated by density gradient ultracentrifugation showed that in the control ApoE-/- mice, VLDL contained apoB-48, apoA-I, and apoA-IV. Diffuse staining in the low molecular weight region also indicated the presence of low molecular weight peptides. Similarly, in mice expressing apoE4-202, VLDL contained apoB-48 apoA-I, apoA-IV and low molecular weight peptides (Fig. 7A). In contrast, in hypertriglyceridemic mice expressing apoE4, only apoB-48 and apoE4 were present. This qualitative picture is consistent with previous studies showing that overexpression of apoE displaces apoCII and other proteins from the lipoprotein particles.

### Rate of VLDL triglyceride production

The rate of VLDL triglyceride production was measured in mice infected with different apoE forms. To determine the effect of the apoE truncations on VLDL triglyceride synthesis, apoE-deficient mice were infected with a dose of 2x10⁹ pfu of AdGFP-E4, 4x10⁹ pfu of AdGFP-E4-259, or 4x10⁹ pfu of the control AdGFP virus. Five days post-infection, when the expression of the apoE transgene was maximum, mice were fasted for four hours and then injected with Triton- WR1339 at a dose of 500 mg/kg of body weight using a 15% solution (w/v) in 0.9% NaCl (Triton-WR1339), which has been shown to completely inhibit VLDL catabolism (Aalto-Setala et al., J. Clin. Invest. 90:1889-1900,1992). Then, serum samples were isolated 5 minutes, 10 minutes, 20 minutes, 30 minutes, 40 minutes, 50 minutes, and 60 minutes after injection with Triton-WR1339. As a control, serum samples were isolated one minute immediately after the injection with the detergent. Serum triglyceride levels were determined as described above, and a linear graph of serum triglyceride versus time was generated. The rate of VLDL-triglyceride secretion expressed in mg/dl/min was calculated from the slope of the linear graph for each individual mouse. Then, the slopes were grouped together and reported in a bar graph as the mean ± standard deviation. Wild-type apoE4 induced a dramatic increase in VLDL triglyceride production compared to that induced by apoE4-259 or control virus (Fig. 12).

To determine the effect of apoE-185 on VLDL triglyceride synthesis and secretion, apoE-deficient mice were infected with a dose of 2x10⁹ pfu of AdGFP-E4, 2x10⁹ pfu of AdGFP, or 1x10¹⁰ pfu of AdGFP-E4-185. The mice were injected with Triton-WR1339 five days following adenoviral infection, as described above. Serum samples were isolated 20 minutes, 40 minutes, and 60 minutes after injection with Triton-WR1339. The rate of triglyceride secretion in mice infected with adenovirus expressing full-length apoE4 was eight-fold higher than the rate of triglyceride secretion in mice infected.with 1 x 10¹⁰ pfu AdGFP-E4-185 (Fig. 19). The rate of VLDL triglyceride secretion was also two-fold higher in mice infected with 2 x 10⁹ pfu of control AdGFP virus than in mice infected with AdGFP-E4-185.

The reduced rate of VLDL triglyceride production in mice expressing apoE4-185 or apoE4-259 compared to the corresponding rate in mice expressing apoE4 suggests that the carboxy terminal region of apoE influences the rate of VLDL triglyceride secretion. This reduced rate of VLDL triglyceride production may contribute to the inability of the truncated apoE mutants to trigger hypertriglyceridemia.

### Purification of apoE Polypeptides

Standard molecular biology techniques can be used to generate fragments or mutants of a native human apoE nucleic acid for use in a suitable expression system (e.g. a prokaryotic expression system, eukaryotic cells, a transgenic animal, or a cell-free system) (Ausubel et al., eds., Current Protocols in Molecular Biology, Vol. 1-3, 1994, John Wiley and Sons, Inc.). For example, recombinant apoE can be expressed in a variety of cells commonly used for recombinant mammalian protein expression, including hamster kidney cells which are available from the American Type Culture Collection (ATCC), Rockville, MD. In particular, apoE2, apoE3, and apoE4 have been expressed in baby hamster kidney (BHK-21) cells from the ATCC (CRL 6282), Cos-1 cells, and a baculovirus expression system (Aleshkov et al., Biochemistry 36:10571-10580). The apoE polypeptides can be harvested and purified from these systems using standard techniques, such as, gel filtration chromatography, ammonium sulfate precipitation, ion-exchange chromatography, hydrophobic interactions chromatography, immuno-affinity chromatography, or polyethylene glycol separation. Additionally the apoE polypeptides can be lyophillized prior to storage, preferably in the presence of albumin, to increase their shelf-life.

The method of Lollar *et al.* for epitope mapping can be used to determine amino acids in the apoE polypeptides that might be immunogenic (*i.e.* cause the production of antibodies to the apoE polypeptide). These amino acids can be changed to alanine to reduce the immunogenicity of the polypeptide (U.S. Patent No. 5,888,974).

Hydrophobic residues of apoE, including Leu261, Thr264, Phe265, Leu268, Val269, Trp276, Leu279, Val280, and Val282, may participate in hydrophobic interactions with lipoprotein particles, resulting in inhibition of lipoprotein lipase. Thus, these residues in apoE can be mutated to polar or charged amino acids to inhibit the induction of hypertriglyceridemia by apoE polypeptides.

### Administration of apoE Polypeptides

It is not intended that the present invention be limited to a particular mode of administration, dosage, or frequency of dosing; the present mode contemplates all modes of administration, including intramuscular, intravenous, intravascular, subcutaneous, and oral. Depending on the dose, the volume of administration can be varied between approximately 0.2 to 2.0 ml/kg body-weight. The preferred dosage of the apoE polypeptide is between 5 and 50 mg/kg body-weight administered in the range of every 8 to 24 hours (in severe cases) to every 2 weeks over a period of at least 6 months, as required. It is to be understood that for any particular subject, specific dosage regimes should be adjusted over time according to the individual need and the professional judgement of the person administering or supervising the administration of the compositions.

The pharmaceutical compositions containing one or more apoE polypeptides can be prepared as described previously in Remingtion's Pharmaceutical Sciences by E. W. Martin. Pharmaceutical stabilizing compounds, delivery vehicles, and/or carrier vehicles may be used. For example, human serum albumin, which has been found to stabilize factor VIII preparations, or other human or animal proteins can be used. Phospholipid vesicles or liposomal suspensions are the preferred pharmaceutically acceptable carriers or delivery vehicles. These can be prepared according to methods known to those skilled in the art.

ApoE polypeptides can be administered with an additive, such as an oil-in-water emulsion with oil as the dispersed phase, a water-in-oil emulsion with oil as the continuous phase, or a dispersion of polar lipids, as described previously for factor VIII (U.S. Patent No. 5,925,739). Dextran, hyaluronic acid, hydrolyzed collagen, hydrolyzed gelatin, poly(0-2-hydroxyethyl) starch, or a soybean emulsion can also be added to increase bioavailability (U.S. Patent No. 5,925,739). The apoE preparation can further contain sodium chloride, calcium chloride, polyethylene glycol, at least 0.01 mg/ml of a polyoxyethylene sorbitan fatty acid ester, or an amino acid in an amount of more than 1 mM (U.S. Patent No. 5,925,739).

### Gene therapy Delivery of apoE Polypeptides

The apoE polypeptids can be delivered by gene therapy using a means such as viral vectors. In this preferred method, an apoE nucleic acid is cloned into the genome of a recombinant virus, such as an adenoviral, an adeno-associated viral, a retroviral, a lentiviral, baculovirus, or a herpes viral vector, as described above. The gene is inserted into the genome of the host cell by viral machinery where it will be expressed by the cell. The viral vector is modified so that it is replication deficient and will not produce virus, preventing viral infection in the host. The general principles for this type of therapy are known to those skilled in the art and have been reviewed in the literature (Kohn et al., Transfusion 29:812-820, 1989). Other possible delivery methods include bone marrow transplantation, direct infection of an artery at the site of an atherosclerotic lesion, and genetic manipulation of a fetus. The cells that have incorporated the apoE nucleic acid can be transplanted directly into a mammal or can be placed in an implantable device, permeable to the encoded apoE polypeptide but impermeable to the cells, that is then transplanted.

For gene delivery using bone marrow transplantation, a subject is exposed to radiation to destroy endogenous apoE-producing bone marrow cells. Donor bone marrow cells are infected *in vitro* with a retrovirus expressing a truncated form of apoE at a multiplicity of infection of approximately 20 - 50 (20 to 50 virus particles per bone marrow cell), and then transplanted into the subject for replacement of the subject's endogenous bone marrow cells with cells producing a truncated form of apoE *in vivo.* This procedure may be performed by one skilled in the art based on the protocol that the National Institutes of Health has established for bone marrow transplantation in cancer patients. For subjects whose endogenous apoE contains mutations associated with Type III hyperlipoproteinemia, which renders apoE-containing VLDL particles resistant to catabolism and results in high serum levels of cholesterol, this procedure may lower serum cholesterol levels. The recombinant bone marrow cells may also produce circulating macrophages that accumulate at sites of atherosclerosis and express the therapeutic truncated apoE protein, resulting in local regression of atherosclerosis.

For the genetic manipulation of a fetus, the nucleus from a one to eight cell stage fetus, an oocyte, or an ovum can be removed and replaced with a nucleus which encodes a truncated form of apoE. This procedure may be performed by one skilled in the art based on the protocols that have been used to clone a variety of mammals, such as cattle, sheep, rabbits, pigs, and mice (see, for example, Prather et al., Biol. Reprod. 37:859-866, 1987; Willadsen, Nature 320:63-65, 1986; Stice and Robl, Biol. Reprod. 39:657-664, 1989; Prather et al., Biol. Reprod. 41:414-418, 1989; Tsunoda et al., J. Exp. Zool. 242:147-151, 1987).

### Administration of apoE Nucleic acids

The apoE nucleic acids used according to the invention can also be administered intravenously or intravascularly. Other possible delivery methods include bone marrow transplantation, direct infection and/or transfection of an artery at the site of an atherosclerotic lesion, and genetic manipulation of a fetus. Preferably the nucleic acids are administered in combination with a liposome and protamine. For any particular subject, the specific dosage regimes should be adjusted over time according to the individual need and the professional judgement of the person administering or supervising the administration of the compositions. A preferred dose is 3 mg of apoE nucleic acid per dl of serum.

### Other Embodiments

While the invention has been described in connection with specific embodiments thereof, it will be understood that it is capable of further modifications and this application is intended to cover any variations, uses, or adaptations of the invention following, in general, the principles of the invention and including such departures from the present disclosure that come within known or customary practice within the art to which the invention pertains and may be applied to the essential features hereinbefore set forth, and follows in the scope of the appended claims.

Other embodiments are within the claims.

## Claims

1. Use of a polypeptide comprising a region of at least 150 amino acids having at least 80% sequence identity to the corresponding region of amino acids 1-259 of a mature, native, human apoE, wherein the polypeptide does not comprise the carboxyl-terminal 260-299 residues of said apoE, for the preparation of a pharmaceutical composition for lowering cholesterol, delaying the onset of atherosclerosis, or treating atherosclerosis in a mammal without inducing hypertriglyceridemia, wherein said polypeptide is to be administered to said mammal and said polypeptide, when administered to or expressed in a mammal, is capable of lowering the total serum cholesterol level without inducing hypertriglyceridemia.

2. The use of claim 1, wherein said polypeptide is to be administered intramuscularly, intravenously, or subcutaneously to said mammal.

3. Use of a nucleic acid encoding a polypeptide comprising a region of at least 150 amino acids having at least 80% sequence identity to the corresponding region of amino acids 1 to 259 of a mature, native, human apoE polypeptide, wherein the nucleic acid does not encode the carboxyl-terminal 260-299 residues of said apoE, for the preparation of a pharmaceutical composition for lowering cholesterol, delaying the onset of atherosclerosis, or regressing atherosclerosis in a mammal without inducing hypertriglyceridemia, wherein said nucleic acid is to be administered to or expressed in said mammal and the nucleic acid, when administered to or expressed in a mammal, lowers the total serum cholesterol level without inducing hypertriglyceridemia.

4. The use of claim 3, wherein said nucleic acid is operably linked to a promoter and contained in an expression vector.

5. The use of claim 3, wherein said nucleic acid is to be intravenously administered to said mammal in combination with a liposome and protamine.

6. The use of claim 3, wherein said nucleic acid is contained in a recombinant viral vector.

7. The use of claim 6, wherein said vector is to be administered intravenously, by bone marrow transplantation or to an artery at the site of a lesion.

8. The use of claim 6, wherein said vector is an adenoviral vector, an adeno-associated viral vector, a lentiviral vector, a herpes viral vector, a retroviral vector or a baculoviral vector.

9. The use of claim 1 or 3, wherein said mammal lacks an endogenous, normally functioning apoE gene, is at risk for developing atherosclerosis due to accumulation of lipoprotein remnants in the bloodstream, has a defect in remnant removal or lacks an endogenous, normally functioning LDL receptor.

10. The use of claim 3, wherein said nucleic acid is to be administered to or expressed in the liver of said mammal.

11. A polypeptide consisting of an amino acid sequence identical to amino acids 1-185, 1-202, 1-229, or 1-259 of a mature, native human apoE of any one of SEQ ID NOs. 1-6 or amino acids 1-203, 1-220, 1-247 or 1-277 of an apoE preprotein of any one of SEQ ID NOs. 14-19.

12. A pharmaceutical composition comprising the polypeptide of claim 11 admixed with a pharmaceutically acceptable carrier substance.

13. A nucleic acid encoding the polypeptide of claim 11.

14. A recombinant DNA molecule comprising the nucleic acid of claim 13 operatively linked to a promoter.

## Patentansprüche

1. Verwendung eines Polypeptids, das eine Region von mindestens 150 Aminosäuren mit mindestens 80% Sequenzidentität zu der korrespondierenden Region der Aminosäuren 1-259 eines reifen, nativen, humanen apoE umfasst, wobei das Polypeptid nicht die carboxyterminalen Reste 260-299 des apoE umfasst, zur Herstellung einer pharmazeutischen Zusammensetzung für eine Absenkung von Cholesterin, eine Verzögerung des Ausbruchs von Atherosklerose oder eine Behandlung von Atherosklerose in einem Säuger ohne eine Induzierung von Hypertriglyceridämie, wobei das Polypeptid an den Säuger zu verabreichen ist und das Polypeptid bei einer Verabreichung an oder Expression in einem Säuger fähig ist, den Gesamtcholesterinspiegel im Serum abzusenken ohne Hypertriglyceridämie zu induzieren.

2. Verwendung nach Anspruch 1, wobei das Polypeptid intramuskulär, intravenös oder subkutan an den Säuger zu verabreichen ist.

3. Verwendung einer Nukleinsäure, die für ein Polypeptid kodiert, das eine Region von mindestens 150 Aminosäuren mit mindestens 80% Sequenzidentität zu der korrespondierenden Region der Aminosäuren 1-259 eines reifen, nativen, humanen apoE-Polypeptids umfasst, wobei die Nukleinsäure nicht für die carboxyterminalen Reste 260-299 des apoE kodiert, zur Herstellung einer pharmazeutischen Zusammensetzung für eine Absenkung von Cholesterin, eine Verzögerung des Ausbruchs von Atherosklerose oder eine Rückentwicklung von Atherosklerose in einem Säuger ohne eine Induzierung von Hypertriglyceridämie, wobei die Nukleinsäure an den Säuger zu verabreichen oder in dem Säuger zu exprimieren ist und die Nukleinsäure bei einer Verabreichung an oder Expression in einem Säuger den Gesamtcholesterinspiegel im Serum senkt ohne Hypertriglyceridämie zu induzieren.

4. Verwendung nach Anspruch 3, wobei die Nukleinsäure mit einem Promotor operabel verbunden ist und in einem Expressionsvektor enthalten ist.

5. Verwendung nach Anspruch 3, wobei die Nukleinsäure intravenös an den Säuger in Kombination mit einem Liposom und Protamin zu verabreichen ist.

6. Verwendung nach Anspruch 3, wobei die Nukleinsäure in einem rekombinanten viralen Vektor enthalten ist.

7. Verwendung nach Anspruch 6, wobei der Vektor intravenös, durch Knochenmarkstransplantation oder an eine Arterie an der Stelle einer Läsion zu verabreichen ist.

8. Verwendung nach Anspruch 6, wobei der Vektor ein adenoviraler Vektor, ein adeno-assoziierter viraler Vektor, ein lentiviraler Vektor, ein herpesviraler Vektor, ein retroviraler Vektor oder ein baculoviraler Vektor ist.

9. Verwendung nach Anspruch 1 oder 3, wobei der Säuger kein endogenes, normal funktionierendes apoE-Gen hat, ein Risiko für eine Entwicklung von Atherosklerose aufgrund einer Anhäufung von Lipoprotein-Überresten in dem Blutstrom aufweist, einen Defekt hinsichtlich einer Beseitigung der Überreste aufweist oder keinen endogenen, normal funktionierenden LDL-Rezeptor aufweist.

10. Verwendung nach Anspruch 3, wobei die Nukleinsäure an die Leber des Säugers zu verabreichen ist oder in der Leber des Säugers zu exprimieren ist.

11. Polypeptid, das aus einer Aminosäuresequenz besteht, die zu den Aminosäuren 1-185, 1-202, 1-229 oder 1-259 eines reifen, nativen humanen apoE gemäß einer der SEQ ID NOs: 1-6 oder den Aminosäuren 1-203, 1-220, 1-247 oder 1-277 eines apoE-Präproteins gemäß einer der SEQ ID NOs: 14-19 identisch ist.

12. Pharmazeutische Zusammensetzung, die das Polypeptid nach Anspruch 11 im Gemisch mit einer pharmazeutisch verträglichen Trägersubstanz umfasst.

13. Nukleinsäure, die für das Polypeptid nach Anspruch 11 kodiert.

14. Rekombinantes DNA-Molekül, das die Nukleinsäure nach Anspruch 13 in operabler Verbindung mit einem Promotor umfasst.

## Revendications

1. Utilisation d'un polypeptide comprenant une région d'au moins 150 acides aminés ayant au moins 80% d'identité de séquence avec la région correspondante des acides aminés 1-259 d'une apoE humaine, native, mature, tandis que le polypeptide ne comprend pas les résidus 260-299 carboxy-terminaux de ladite apoE, pour la préparation d'une composition pharmaceutique pour abaisser le cholestérol, retarder l'apparition d'athérosclérose, ou traiter une athérosclérose chez un mammifère sans induire une hypertriglycéridémie, tandis que ledit polypeptide est destiné à être administré au dit mammifère et que ledit polypeptide, lorsqu'il est administré à ou exprimé dans un mammifère, est capable d'abaisser le taux de cholestérol sérique total sans induire une hypertriglycéridémie.

2. Utilisation selon la revendication 1, dans laquelle ledit polypeptide est destiné à être administré par voie intramusculaire, intraveineuse, ou sous-cutanée au dit mammifère.

3. Utilisation d'un acide nucléique codant pour un polypeptide comprenant une région d'au moins 150 acides aminés ayant au moins 80% d'identité de séquence avec la région correspondante des acides aminés 1 à 259 d'un polypeptide d'apoE humaine, native, mature, tandis que l'acide nucléique ne code pas pour les résidus 260-299 carboxy-terminaux de ladite apoE, pour la préparation d'une composition pharmaceutique pour abaisser le cholestérol, retarder l'apparition d'athérosclérose, ou faire régresser une athérosclérose chez un mammifère sans induire une hypertriglycéridémie, tandis que ledit acide nucléique est destiné à être administré au dit ou exprimé dans ledit mammifère et que l'acide nucléique, lorsqu'il est administré à ou exprimé dans un mammifère, abaisse le taux de cholestérol sérique total sans induire une hypertriglycéridémie.

4. Utilisation selon la revendication 3, dans laquelle ledit acide nucléique est lié de manière opérationnelle à un promoteur et contenu dans un vecteur d'expression.

5. Utilisation selon la revendication 3, dans laquelle ledit acide nucléique est destiné à être administré par voie intraveineuse au dit mammifère en combinaison avec un liposome et une protamine.

6. Utilisation selon la revendication 3, dans laquelle ledit acide nucléique est contenu dans un vecteur viral recombinant.

7. Utilisation selon la revendication 6, dans laquelle ledit vecteur est destiné à être administré par voie intraveineuse, par transplantation de moelle osseuse ou dans une artère sur le site d'une lésion.

8. Utilisation selon la revendication 6, dans laquelle ledit vecteur est un vecteur adénoviral, un vecteur viral adéno-associé, un vecteur lentiviral, un vecteur viral d'herpès, un vecteur rétroviral ou un vecteur baculoviral.

9. Utilisation selon la revendication 1 ou 3, dans laquelle ledit mammifère est dénué de gène apoE endogène, fonctionnant normalement, est à risque pour le développement d'une athérosclérose due à l'accumulation de reliquats de lipoprotéine dans la circulation sanguine, présente un défaut dans l'élimination du reliquat ou est dénué de récepteur de LDL endogène, fonctionnant normalement.

10. Utilisation selon la revendication 3, dans laquelle ledit acide nucléique est destiné à être administré à ou exprimé dans le foie du dit mammifère.

11. Polypeptide consistant en une séquence d'acides aminés identique aux acides aminés 1-185, 1-202, 1-229 ou 1-259 d'une apoE humaine native, mature selon l'une quelconque des SEQ ID NOs. 1-6 ou aux acides aminés 1-203, 1-220, 1-247 ou 1-277 d'une préprotéine d'apoE selon l'une quelconque des SEQ ID NOs.14-19.

12. Composition pharmaceutique comprenant le polypeptide selon la revendication 11, en mélange avec une substance support pharmaceutiquement acceptable.

13. Acide nucléique codant pour le polypeptide selon la revendication 11.

14. Molécule d'ADN recombinant comprenant l'acide nucléique selon la revendication 13 lié de manière opérationnelle à un promoteur.


### SEQUENCE LISTING

<110> KOS Pharmaceuticals, Inc., Trustees of Boston University
<120> Compounds and methods for lowering cholesterol levels without inducing hypertriglyceridemia
<130> 171-127
<140> EP 01926716.0
   <141> 2001-04-06
<150> US 09/544,386
   <151> 2000-04-06
<150> US 09/679,088
   <151> 2000-10-04
<150> US 09//827,854
   <151> 2001-04-05
<160> 19
<170> FastSEQ for Windows Version 4.0
<210> 1
   <211> 299
   <212> PRT
   <213> Homo sapiens
<400> 1
   Lys Val Glu Gln Ala Val Glu Thr Glu Pro Glu Pro Glu Leu Arg Gln
   1               5                   10                  15
   Gln Thr Glu Trp Gln Ser Gly Gln Arg Trp Glu Leu Ala Leu Gly Arg
               20                  25                  30
   Phe Trp Asp Tyr Leu Arg Trp Val Gln Thr Leu Ser Glu Gln Val Gln
           35                  40                  45
   Glu Glu Leu Leu Ser Ser Gln Val Thr Gln Glu Leu Arg Ala Leu Met
       50                  55                  60
   Asp Glu Thr Met Lys Glu Leu Lys Ala Tyr Lys Ser Glu Leu Glu Glu
   65                  70                  75                  80
   Gln Leu Thr Pro Val Ala Glu Glu Thr Arg Ala Arg Leu Ser Lys Glu
                   85                  90                  95
   Leu Gln Ala Ala Gln Ala Arg Leu Gly Ala Asp Met Glu Asp Val Arg
               100                 105                 110
   Gly Arg Leu Val Gln Tyr Arg Gly Glu Val Gln Ala Met Leu Gly Gln
           115                 120                 125
   Ser Thr Glu Glu Leu Arg Val Arg Leu Ala Ser His Leu Arg Lys Leu
       130                 135                 140
   Arg Lys Arg Leu Leu Arg Asp Ala Asp Asp Leu Gln Lys Arg Leu Ala
   145                 150                 155                 160
   Val Tyr Gln Ala Gly Ala Arg Glu Gly Ala Glu Arg Gly Leu Ser Ala
                   165                 170                 175
   Ile Arg Glu Arg Leu Gly Pro Leu Val Glu Gln Gly Arg Val Arg Ala
               180                 185                 190
   Ala Thr Val Gly Ser Leu Ala Gly Gln Pro Leu Gln Glu Arg Ala Gln
           195                 200                 205
   Ala Trp Gly Glu Arg Leu Arg Ala Arg Met Glu Glu Met Gly Ser Arg
       210                 215                 220
   Thr Arg Asp Arg Leu Asp Glu Val Lys Glu Gln Val Ala Glu Val Arg
   225                 230                 235                 240
   Ala Lys Leu Glu Glu Gln Ala Gln Gln Ile Arg Leu Gln Ala Glu Ala
                   245                 250                 255
   Phe Gln Ala Arg Leu Lys Ser Trp Phe Glu Pro Leu Val Glu Asp Met
               260                 265                 270
   Gln Arg Gln Trp Ala Gly Leu Val Glu Lys Val Gln Ala Ala Val Gly
           275                 280                 285
   Thr Ser Ala Ala Pro Val Pro Ser Asp Asn His
       290                 295
<210> 2
   <211> 299
   <212> PRT
   <213> Homo sapiens
<400> 2
   Lys Val Glu Gln Ala Val Glu Thr Glu Pro Glu Pro Glu Leu Arg Gln
    1              5                   10                  15
   Gln Thr Glu Trp Gln Ser Gly Gln Arg Trp Glu Leu Ala Leu Gly Arg
               20                  25                  30
   Phe Trp Asp Tyr Leu Arg Trp Val Gln Thr Leu Ser Glu Gln Val Gln
           35                  40                  45
   Glu Glu Leu Leu Ser Ser Gln Val Thr Gln Glu Leu Arg Ala Leu Met
       50                  55                  60
   Asp Glu Thr Met Lys Glu Leu Lys Ala Tyr Lys Ser Glu Leu Glu Glu
   65                  70                  75                  80
   Gln Leu Thr Pro Val Ala Glu Glu Thr Arg Ala Arg Leu Ser Lys Glu
                   85                  90                  95
   Leu Gln Ala Ala Gln Ala Arg Leu Gly Ala Asp Met Glu Asp Val Cys
               100                 105                 110
   Gly Arg Leu Val Gln Tyr Arg Gly Glu Val Gln Ala Met Leu Gly Gln
           115                 120                 125
   Ser Thr Glu Glu Leu Arg Val Arg Leu Ala Ser His Leu Arg Lys Leu
       130                 135                 140
   Arg Lys Arg Leu Leu Arg Asp Ala Asp Asp Leu Gln Lys Arg Leu Ala
   145                 150                 155                 160
   Val Tyr Gln Ala Gly Ala Arg Glu Gly Ala Glu Arg Gly Leu Ser Ala
                   165                 170                 175
   Ile Arg Glu Arg Leu Gly Pro Leu Val Glu Gln Gly Arg Val Arg Ala
               180                 185                 190
   Ala Thr Val Gly Ser Leu Ala Gly Gln Pro Leu Gln Glu Arg Ala Gln
           195                 200                 205
   Ala Trp Gly Glu Arg Leu Arg Ala Arg Met Glu Glu Met Gly Ser Arg
       210                 215                 220
   Thr Arg Asp Arg Leu Asp Glu Val Lys Glu Gln Val Ala Glu Val Arg
   225                 230                 235                 240
   Ala Lys Leu Glu Glu Gln Ala Gln Gln Ile Arg Leu Gln Ala Glu Ala
                   245                 250                 255
   Phe Gln Ala Arg Leu Lys Ser Trp Phe Glu Pro Leu Val Glu Asp Met
               260                 265                 270
   Gln Arg Gln Trp Ala Gly Leu Val Glu Lys Val Gln Ala Ala Val Gly
           275                 280                 285
   Thr Ser Ala Ala Pro Val Pro Ser Asp Asn His
       290                 295
<210> 3
   <211> 299
   <212> PRT
   <213> Homo sapiens
<400> 3
   Lys Val Glu Gln Ala Val Glu Thr Glu Pro Glu Pro Glu Leu Arg Gln
    1              5                   10                  15
   Gln Thr Glu Trp Gln Ser Gly Gln Arg Trp Glu Leu Ala Leu Gly Arg
   20 25 30
   Phe Trp Asp Tyr Leu Arg Trp Val Gln Thr Leu Ser Glu Gln Val Gln
   35 40 45
   Glu Glu Leu Leu Ser Ser Gln Val Thr Gln Glu Leu Arg Ala Leu Met
   50 55 60
   Asp Glu Thr Met Lys Glu Leu Lys Ala Tyr Lys Ser Glu Leu Glu Glu
   65 70 75 80
   Gln Leu Thr Pro Val Ala Glu Glu Thr Arg Ala Arg Leu Ser Lys Glu
   85 90 95
   Leu Gln Ala Ala Gln Ala Arg Leu Gly Ala Asp Met Glu Asp Val Cys
   100 105 110
   Gly Arg Leu Val Gln Tyr Arg Gly Glu Val Gln Ala Met Leu Gly Gln
   115 120 125
   Ser Thr Glu Glu Leu Arg Val Arg Leu Ala Ser His Leu Arg Lys Leu
   130 135 140
   Arg Lys Arg Leu Leu Arg Asp Ala Asp Asp Leu Gln Lys Cys Leu Ala
   145 150 155 160
   Val Tyr Gln Ala Gly Ala Arg Glu Gly Ala Glu Arg Gly Leu Ser Ala
   165 170 175
   Ile Arg Glu Arg Leu Gly Pro Leu Val Glu Gln Gly Arg Val Arg Ala
   180 185 190
   Ala Thr Val Gly Ser Leu Ala Gly Gln Pro Leu Gln Glu Arg Ala Gln
   195 200 205
   Ala Trp Gly Glu Arg Leu Arg Ala Arg Met Glu Glu Met Gly Ser Arg
   210 215 220
   Thr Arg Asp Arg Leu Asp Glu Val Lys Glu Gln Val Ala Glu Val Arg
   225 230 235 240
   Ala Lys Leu Glu Glu Gln Ala Gln Gln Ile Arg Leu Gln Ala Glu Ala
   245 250 255
   Phe Gln Ala Arg Leu Lys Ser Trp Phe Glu Pro Leu Val Glu Asp Met
   260 265 270
   Gln Arg Gln Trp Ala Gly Leu Val Glu Lys Val Gln Ala Ala Val Gly
   275 280 285
   Thr Ser Ala Ala Pro Val Pro Ser Asp Asn His
   290 295
<210> 4
   <211> 299
   <212> PRT
   <213> Homo sapiens
<400> 4
<210> 5
   <211> 299
   <212> PRT
   <213> Homo sapiens
<400> 5
   Lys Val Glu Gln Ala Val Glu Thr Glu Pro Glu Pro Glu Leu Arg Gln
   1               5                   10                  15
   Gln Thr Glu Trp Gln Ser Gly Gln Arg Trp Glu Leu Ala Leu Gly Arg
               20                  25                  30
   Phe Trp Asp Tyr Leu Arg Trp Val Gln Thr Leu Ser Glu Gln Val Gln
           35                  40                  45
   Glu Glu Leu Leu Ser Ser Gln Val Thr Gln Glu Leu Arg Ala Leu Met
       50                  55                  60
   Asp Glu Thr Met Lys Glu Leu Lys Ala Tyr Lys Ser Glu Leu Glu Glu
   65                  70                  75                  80
   Gln Leu Thr Pro Val Ala Glu Glu Thr Arg Ala Arg Leu Ser Lys Glu
                   85                  90                  95
   Leu Gln Ala Ala Gln Ala Arg Leu Gly Ala Asp Met Glu Asp Val Cys
               100                 105                 110
   Gly Arg Leu Val Gln Tyr Arg Gly Glu Val Gln Ala Met Leu Gly Gln
           115                 120                 125
   Ser Thr Glu Glu Leu Arg Val Arg Leu Ala Ser His Leu Arg Lys Leu
       130                 135                 140
   Cys Lys Arg Leu Leu Arg Asp Ala Asp Asp Leu Gln Lys Arg Leu Ala
   145                 150                 155                 160
   Val Tyr Gln Ala Gly Ala Arg Glu Gly Ala Glu Arg Gly Leu Ser Ala
                   165                 170                 175
   Ile Arg Glu Arg Leu Gly Pro Leu Val Glu Gln Gly Arg Val Arg Ala
               180                 185                 190
   Ala Thr Val Gly Ser Leu Ala Gly Gln Pro Leu Gln Glu Arg Ala Gln
           195                 200                 205
   Ala Trp Gly Glu Arg Leu Arg Ala Arg Met Glu Glu Met Gly Ser Arg
       210                 215                 220
   Thr Arg Asp Arg Leu Asp Glu Val Lys Glu Gln Val Ala Glu Val Arg
   225                 230                 235                 240
   Ala Lys Leu Glu Glu Gln Ala Gln Gln Ile Arg Leu Gln Ala Glu Ala
                   245                 250                 255
   Phe Gln Ala Arg Leu Lys Ser Trp Phe Glu Pro Leu Val Glu Asp Met
               260                 265                 270
   Gln Arg Gln Trp Ala Gly Leu Val Glu Lys Val Gln Ala Ala Val Gly
           275                 280                 285
   Thr Ser Ala Ala Pro Val Pro Ser Asp Asn His
       290                 295
<210> 6
   <211> 299
   <212> PRT
   <213> Homo sapiens
<400> 6
   Lys Val Glu Gln Ala Val Glu Thr Glu Pro Glu Pro Glu Leu Arg Gln
   1               5                   10                  15
   Gln Thr Glu Trp Gln Ser Gly Gln Arg Trp Glu Leu Ala Leu Gly Arg
               20                  25                  30
   Phe Trp Asp Tyr Leu Arg Trp Val Gln Thr Leu Ser Glu Gln Val Gln
           35                  40                  45
   Glu Glu Leu Leu Ser Ser Gln Val Thr Gln Glu Leu Arg Ala Leu Met
       50                  55                  60
   Asp Glu Thr Met Lys Glu Leu Lys Ala Tyr Lys Ser Glu Leu Glu Glu
   65                  70                  75                  80
   Gln Leu Thr Pro Val Ala Glu Glu Thr Arg Ala Arg Leu Ser Lys Glu
                   85                  90                  95
   Leu Gln Ala Ala Gln Ala Arg Leu Gly Ala Asp Met Glu Asp Val Cys
               100                 105                 110
   Gly Arg Leu Val Gln Tyr Arg Gly Glu Val Gln Ala Met Leu Gly Gln
           115                 120                 125
   Ser Thr Glu Glu Leu Arg Val Arg Leu Ala Ser His Leu Arg Lys Leu
       130                 135                 140
   Arg Gln Arg Leu Leu Arg Asp Ala Asp Asp Leu Gln Lys Arg Leu Ala
   145                 150                 155                 160
   Val Tyr Gln Ala Gly Ala Arg Glu Gly Ala Glu Arg Gly Leu Ser Ala
                   165                 170                 175
   Ile Arg Glu Arg Leu Gly Pro Leu Val Glu Gln Gly Arg Val Arg Ala
               180                 185                 190
   Ala Thr Val Gly Ser Leu Ala Gly Gln Pro Leu Gln Glu Arg Ala Gln
   Ala Trp Gly Glu Arg Leu Arg Ala Arg Met Glu Glu Met Gly Ser Arg
       210                 215                 220
   Thr Arg Asp Arg Leu Asp Glu Val Lys Glu Gln Val Ala Glu Val Arg
   225                 230                 235                 240
   Ala Lys Leu Glu Glu Gln Ala Gln Gln Ile Arg Leu Gln Ala Glu Ala
                   245                 250                 255
   Phe Gln Ala Arg Leu Lys Ser Trp Phe Glu Pro Leu Val Glu Asp Met
               260                 265                 270
   Gln Arg Gln Trp Ala Gly Leu Val Glu Lys Val Gln Ala Ala Val Gly
           275                 280                 285
   Thr Ser Ala Ala Pro Val Pro Ser Asp Asn His
       290                 295
<210> 7
   <211> 1156
   <212> DNA
   <213> Homo sapiens
<400> 7
   cgcagcggag gtgaaggacg tccttcccca ggagccgact ggccaatcac aggcaggaag 60
   atgaaggttc tgtgggctgc gttgctggtc acattcctgg caggatgcca ggccaaggtg 120
   gagcaagcgg tggagacaga gccggagccc gagctgcgcc agcagaccga gtggcagagc 180
   ggccagcgct gggaactggc actgggtcgc ttttgggatt acctgcgctg ggtgcagaca 240
   ctgtctgagc aggtgcagga ggagctgctc agctcccagg tcacccagga actgagggcg 300
   ctgatggacg agaccatgaa ggagttgaag gcctacaaat cggaactgga ggaacaactg 360
   accccggtgg cggaggagac gcgggcacgg ctgtccaagg agctgcaggc ggcgcaggcc 420
   cggctgggcg cggacatgga ggacgtgcgc ggccgcctgg tgcagtaccg cggcgaggtg 480
   caggccatgc tcggccagag caccgaggag ctgcgggtgc gcctcgcctc ccacctgcgc 540
   aagctgcgta agcggctcct ccgcgatgcc gatgacctgc agaagcgcct ggcagtgtac 600
   caggccgggg cccgcgaggg cgccgagcgc ggcctcagcg ccatccgcga gcgcctgggg 660
   cccctggtgg aacagggccg cgtgcgggcc gccactgtgg gctccctggc cggccagccg 720
   ctacaggagc gggcccaggc ctggggcgag cggctgcgcg cgcggatgga ggagatgggc 780
   agccggaccc gcgaccgcct ggacgaggtg aaggagcagg tggcggaggt gcgcgccaag 840
   ctggaggagc aggcccagca gatacgcctg caggccgagg ccttccaggc ccgcctcaag 900
   agctggttcg agcccctggt ggaagacatg cagcgccagt gggccgggct ggtggagaag 960
   gtgcaggctg ccgtgggcac cagcgccgcc cctgtgccca gcgacaatca ctgaacgccg 1020
   aagcctgcag ccatgcgacc ccacgccacc ccgtgcctcc tgcctccgcg cagcctgcag 1080
   cgggagaccc tgtccccgcc ccagccgtcc tcctggggtg gaccctagtt taataaagat 1140
   tcaccaagtt tcacgc                                                 1156
<210> 8
   <211> 1156
   <212> DNA
   <213> Homo sapiens
<400> 8
   cgcagcggag gtgaaggacg tccttcccca ggagccgact ggccaatcac aggcaggaag 60
   atgaaggttc tgtgggctgc gttgctggtc acattcctgg caggatgcca ggccaaggtg 120
   gagcaagcgg tggagacaga gccggagccc gagctgcgcc agcagaccga gtggcagagc 180
   ggccagcgct gggaactggc actgggtcgc ttttgggatt acctgcgctg ggtgcagaca 240
   ctgtctgagc aggtgcagga ggagctgctc agctcccagg tcacccagga actgagggcg 300
   ctgatggacg agaccatgaa ggagttgaag gcctacaaat cggaactgga ggaacaactg 360
   accccggtgg cggaggagac gcgggcacgg ctgtccaagg agctgcaggc ggcgcaggcc 420
   cggctgggcg cggacatgga ggacgtgtgc ggccgcctgg tgcagtaccg cggcgaggtg 480
   caggccatgc tcggccagag caccgaggag ctgcgggtgc gcctcgcctc ccacctgcgc 540
   aagctgcgta agcggctcct ccgcgatgcc gatgacctgc agaagcgcct ggcagtgtac 600
   caggccgggg cccgcgaggg cgccgagcgc ggcctcagcg ccatccgcga gcgcctgggg 660
   cccctggtgg aacagggccg cgtgcgggcc gccactgtgg gctccctggc cggccagccg 720
   ctacaggagc gggcccaggc ctggggcgag cggctgcgcg cgcggatgga ggagatgggc 780
   agccggaccc gcgaccgcct ggacgaggtg aaggagcagg tggcggaggt gcgcgccaag 840
   ctggaggagc aggcccagca gatacgcctg caggccgagg ccttccaggc ccgcctcaag 900
   agctggttcg agcccctggt ggaagacatg cagcgccagt gggccgggct ggtggagaag 960
   gtgcaggctg ccgtgggcac cagcgccgcc cctgtgccca gcgacaatca ctgaacgccg 1020
   aagcctgcag ccatgcgacc ccacgccacc ccgtgcctcc tgcctccgcg cagcctgcag 1080
   cgggagaccc tgtccccgcc ccagccgtcc tcctggggtg gaccctagtt taataaagat 1140
   tcaccaagtt tcacgc                                                 1156
<210> 9
   <211> 1156
   <212> DNA
   <213> Homo sapiens
<400> 9
   cgcagcggag gtgaaggacg tccttcccca ggagccgact ggccaatcac aggcaggaag 60
   atgaaggttc tgtgggctgc gttgctggtc acattcctgg caggatgcca ggccaaggtg 120
   gagcaagcgg tggagacaga gccggagccc gagctgcgcc agcagaccga gtggcagagc 180
   ggccagcgct gggaactggc actgggtcgc ttttgggatt acctgcgctg ggtgcagaca 240
   ctgtctgagc aggtgcagga ggagctgctc agctcccagg tcacccagga actgagggcg 300
   ctgatggacg agaccatgaa ggagttgaag gcctacaaat cggaactgga ggaacaactg 360
   accccggtgg cggaggagac gcgggcacgg ctgtccaagg agctgcaggc ggcgcaggcc 420
   cggctgggcg cggacatgga ggacgtgtgc ggccgcctgg tgcagtaccg cggcgaggtg 480
   caggccatgc tcggccagag caccgaggag ctgcgggtgc gcctcgcctc ccacctgcgc 540
   aagctgcgta agcggctcct ccgcgatgcc gatgacctgc agaagtgcct ggcagtgtac 600
   caggccgggg cccgcgaggg cgccgagcgc ggcctcagcg ccatccgcga gcgcctgggg 660
   cccctggtgg aacagggccg cgtgcgggcc gccactgtgg gctccctggc cggccagccg 720
   ctacaggagc gggcccaggc ctggggcgag cggctgcgcg cgcggatgga ggagatgggc 780
   agccggaccc gcgaccgcct ggacgaggtg aaggagcagg tggcggaggt gcgcgccaag 840
   ctggaggagc aggcccagca gatacgcctg caggccgagg ccttccaggc ccgcctcaag 900
   agctggttcg agcccctggt ggaagacatg cagcgccagt gggccgggct ggtggagaag 960
   gtgcaggctg ccgtgggcac cagcgccgcc cctgtgccca gcgacaatca ctgaacgccg 1020
   aagcctgcag ccatgcgacc ccacgccacc ccgtgcctcc tgcctccgcg cagcctgcag 1080
   cgggagaccc tgtccccgcc ccagccgtcc tcctggggtg gaccctagtt taataaagat 1140
   tcaccaagtt tcacgc                                                 1156
<210> 10
   <211> 1156
   <212> DNA
   <213> Homo sapiens
<400> 10
   cgcagcggag gtgaaggacg tccttcccca ggagccgact ggccaatcac aggcaggaag 60
   atgaaggttc tgtgggctgc gttgctggtc acattcctgg caggatgcca ggccaaggtg 120
   gagcaagcgg tggagacaga gccggagccc gagctgcgcc agcagaccga gtggcagagc 180
   ggccagcgct gggaactggc actgggtcgc ttttgggatt acctgcgctg ggtgcagaca 240
   ctgtctgagc aggtgcagga ggagctgctc agctcccagg tcacccagga actgagggcg 300
   ctgatggacg agaccatgaa ggagttgaag gcctacaaat cggaactgga ggaacaactg 360
   accccggtgg cggaggagac gcgggcacgg ctgtccaagg agctgcaggc ggcgcaggcc 420
   cggctgggcg cggacatgga ggacgtgtgc ggccgcctgg tgcagtaccg cggcgaggtg 480
   caggccatgc tcgaccagag caccgaggag ctgcgggtgc gcctcgcctc ccacctgcgc 540
   aagctgcgta agcggctcct ccgcgatgcc gatgacctgc agaagtgcct ggcagtgtac 600
   caggccgggg cccgcgaggg cgccgagcgc ggcctcagcg ccatccgcga gcgcctgggg 660
   cccctggtgg aacagggccg cgtgcgggcc gccactgtgg gctccctggc cggccagccg 720
   ctacaggagc gggcccaggc ctggggcgag cggctgcgcg cgcggatgga ggagatgggc 780
   agccggaccc gcgaccgcct ggacgaggtg aaggagcagg tggcggaggt gcgcgccaag 840
   ctggaggagc aggcccagca gatacgcctg caggccgagg ccttccaggc ccgcctcaag 900
   agctggttcg agcccctggt ggaagacatg cagcgccagt gggccgggct ggtggagaag 960
   gtgcaggctg ccgtgggcac cagcgccgcc cctgtgccca gcgacaatca ctgaacgccg 1020
   aagcctgcag ccatgcgacc ccacgccacc ccgtgcctcc tgcctccgcg cagcctgcag 1080
   cgggagaccc tgtccccgcc ccagccgtcc tcctggggtg gaccctagtt taataaagat 1140
   tcaccaagtt tcacgc                                                 1156
<210> 11
   <211> 1156
   <212> DNA
   <213> Homo sapiens
<400> 11
<210> 12
   <211> 1156
   <212> DNA
   <213> Homo sapiens
<400> 12
   cgcagcggag gtgaaggacg tccttcccca ggagccgact ggccaatcac aggcaggaag 60
   atgaaggttc tgtgggctgc gttgctggtc acattcctgg caggatgcca ggccaaggtg 120
   gagcaagcgg tggagacaga gccggagccc gagctgcgcc agcagaccga gtggcagagc 180
   ggccagcgct gggaactggc actgggtcgc ttttgggatt acctgcgctg ggtgcagaca 240
   ctgtctgagc aggtgcagga ggagctgctc agctcccagg tcacccagga actgagggcg 300
   ctgatggacg agaccatgaa ggagttgaag gcctacaaat cggaactgga ggaacaactg 360
   accccggtgg cggaggagac gcgggcacgg ctgtccaagg agctgcaggc ggcgcaggcc 420
   cggctgggcg cggacatgga ggacgtgtgc ggccgcctgg tgcagtaccg cggcgaggtg 480
   caggccatgc tcggccagag caccgaggag ctgcgggtgc gcctcgcctc ccacctgcgc 540
   aagctgcgtc agcggctcct ccgcgatgcc gatgacctgc agaagcgcct ggcagtgtac 600
   caggccgggg cccgcgaggg cgccgagcgc ggcctcagcg ccatccgcga gcgcctgggg 660
   cccctggtgg aacagggccg cgtgcgggcc gccactgtgg gctccctggc cggccagccg 720
   ctacaggagc gggcccaggc ctggggcgag cggctgcgcg cgcggatgga ggagatgggc 780
   agccggaccc gcgaccgcct ggacgaggtg aaggagcagg tggcggaggt gcgcgccaag 840
   ctggaggagc aggcccagca gatacgcctg caggccgagg ccttccaggc ccgcctcaag 900
   agctggttcg agcccctggt ggaagacatg cagcgccagt gggccgggct ggtggagaag 960
   gtgcaggctg ccgtgggcac cagcgccgcc cctgtgccca gcgacaatca ctgaacgccg 1020
   aagcctgcag ccatgcgacc ccacgccacc ccgtgcctcc tgcctccgcg cagcctgcag 1080
   cgggagaccc tgtccccgcc ccagccgtcc tcctggggtg gaccctagtt taataaagat 1140
   tcaccaagtt tcacgc                                                 1156
<210> 13
   <211> 18
   <212> PRT
   <213> Homo sapiens
<400> 13
   Met Lys Val Leu Trp Ala Ala Leu Leu Val Thr Phe Leu Ala Gly Cys
    1               5                  10                  15
   Gln Ala
<210> 14
   <211> 317
   <212> PRT
   <213> Homo sapiens
<400> 14
<210> 15
   <211> 317
   <212> PRT
   <213> Homo sapiens
<400> 15
<210> 16
   <211> 317
   <212> PRT
   <213> Homo sapiens
<400> 16
<210> 17
   <211> 317
   <212> PRT
   <213> Homo sapiens
<400> 17
   Met Lys Val Leu Trp Ala Ala Leu Leu Val Thr Phe Leu Ala Gly Cys
    1               5                  10                  15
   Gln Ala Lys Val Glu Gln Ala Val Glu Thr Glu Pro Glu Pro Glu Leu
               20                  25                  30
   Arg Gln Gln Thr Glu Trp Gln Ser Gly Gln Arg Trp Glu Leu Ala Leu
           35                  40                  45
   Gly Arg Phe Trp Asp Tyr Leu Arg Trp Val Gln Thr Leu Ser Glu Gln
           50                  55                  60
   Val Gln Glu Glu Leu Leu Ser Ser Gln Val Thr Gln Glu Leu Arg Ala
   65                  70                  75                  80
   Leu Met Asp Glu Thr Met Lys Glu Leu Lys Ala Tyr Lys Ser Glu Leu
                   85                  90                  95
   Glu Glu Gln Leu Thr Pro Val Ala Glu Glu Thr Arg Ala Arg Leu Ser
               100                 105                 110
   Lys Glu Leu Gln Ala Ala Gln Ala Arg Leu Gly Ala Asp Met Glu Asp
           115                 120                 125
   Val Cys Gly Arg Leu Val Gln Tyr Arg Gly Glu Val Gln Ala Met Leu
       130                 135                 140
   Asp Gln Ser Thr Glu Glu Leu Arg Val Arg Leu Ala Ser His Leu Arg
   145                 150                 155                 160
   Lys Leu Arg Lys Arg Leu Leu Arg Asp Ala Asp Asp Leu Gln Lys Cys
                   165                 170                 175
   Leu Ala Val Tyr Gln Ala Gly Ala Arg Glu Gly Ala Glu Arg Gly Leu
               180                 185                 190
   Ser Ala Ile Arg Glu Arg Leu Gly Pro Leu Val Glu Gln Gly Arg Val
           195                 200                 205
   Arg Ala Ala Thr Val Gly Ser Leu Ala Gly Gln Pro Leu Gln Glu Arg
       210                 215                 220
   Ala Gln Ala Trp Gly Glu Arg Leu Arg Ala Arg Met Glu Glu Met Gly
   225                 230                 235                 240
   Ser Arg Thr Arg Asp Arg Leu Asp Glu Val Lys Glu Gln Val Ala Glu
                   245                 250                 255
   Val Arg Ala Lys Leu Glu Glu Gln Ala Gln Gln Ile Arg Leu Gln Ala
               260                 265                 270
   Glu Ala Phe Gln Ala Arg Leu Lys Ser Trp Phe Glu Pro Leu Val Glu
           275                 280                 285
   Asp Met Gln Arg Gln Trp Ala Gly Leu Val Glu Lys Val Gln Ala Ala
       290                 295                 300
   Val Gly Thr Ser Ala Ala Pro Val Pro Ser Asp Asn His
   305                 310                 315
<210> 18
   <211> 317
   <212> PRT
   <213> Homo sapiens
<400> 18
<210> 19
   <211> 317
   <212> PRT
   <213> Homo sapiens
<400> 19
   Met Lys Val Leu Trp Ala Ala Leu Leu Val Thr Phe Leu Ala Gly Cys
   1 5 10 15
   Gln Ala Lys Val Glu Gln Ala Val Glu Thr Glu Pro Glu Pro Glu Leu
   20 25 30
   Arg Gln Gln Thr Glu Trp Gln Ser Gly Gln Arg Trp Glu Leu Ala Leu
   35 40 45
   Gly Arg Phe Trp Asp Tyr Leu Arg Trp Val Gln Thr Leu Ser Glu Gln
   50 55 60
   Val Gln Glu Glu Leu Leu Ser Ser Gln Val Thr Gln Glu Leu Arg Ala
   65 70 75 80
   Leu Met Asp Glu Thr Met Lys Glu Leu Lys Ala Tyr Lys Ser Glu Leu
   85 90 95
   Glu Glu Gln Leu Thr Pro Val Ala Glu Glu Thr Arg Ala Arg Leu Ser
   100 105 110
   Lys Glu Leu Gln Ala Ala Gln Ala Arg Leu Gly Ala Asp Met Glu Asp
   115 120 125
   Val Cys Gly Arg Leu Val Gln Tyr Arg Gly Glu Val Gln Ala Met Leu
   130 135 140
   Gly Gln Ser Thr Glu Glu Leu Arg Val Arg Leu Ala Ser His Leu Arg
   145 150 155 160
   Lys Leu Arg Gln Arg Leu Leu Arg Asp Ala Asp Asp Leu Gln Lys Arg
   165 170 175
   Leu Ala Val Tyr Gln Ala Gly Ala Arg Glu Gly Ala Glu Arg Gly Leu
   180 185 190
   Ser Ala Ile Arg Glu Arg Leu Gly Pro Leu Val Glu Gln Gly Arg Val
   195 200 205
   Arg Ala Ala Thr Val Gly Ser Leu Ala Gly Gln Pro Leu Gln Glu Arg
   210 215 220
   Ala Gln Ala Trp Gly Glu Arg Leu Arg Ala Arg Met Glu Glu Met Gly
   225 230 235 240
   Ser Arg Thr Arg Asp Arg Leu Asp Glu Val Lys Glu Gln Val Ala Glu
   245 250 255
   Val Arg Ala Lys Leu Glu Glu Gln Ala Gln Gln Ile Arg Leu Gln Ala
   260 265 270
   Glu Ala Phe Gln Ala Arg Leu Lys Ser Trp Phe Glu Pro Leu Val Glu
   275 280 285
   Asp Met Gln Arg Gln Trp Ala Gly Leu Val Glu Lys Val Gln Ala Ala
   290 295 300
   Val Gly Thr Ser Ala Ala Pro Val Pro Ser Asp Asn His
   305 310 315